# FASCICULE DE BREVET EUROPEEN

(11) **EP 0 556 111 B1**
(45) Date de publication et mention de la délivrance du brevet: **04.07.2001**
(21) Numéro de dépôt: 93400323.7
(22) Date de dépôt: 09.02.1993
(51) Int. Cl.: C12N 15/62, C12N 15/70, C12N 1/21, C07K 14/00, G01N 33/577, A61K 39/395

(54) **Protéines hybrides complexes, leur procédé de préparation, et leurs applications comme agent de diagnostic, comme agent à visée thérapeutique ou comme reactif utilisable en imagerie medicale**
Hybride Proteinkomplexe, betreffendes Herstellungsverfahren, und ihre Anwendungen in der Diagnostik, als therapeutische Agens oder Reaktionsmittel, das in der Medizin Verwendung findet
Hybrid protein complexes, their process of preparation, and their applications as an agent in diagnostic and in the therapeutic field or as a reagent relevant in medical approaches

(30) Priorité: 11.02.1992 FR 9201505
(43) Date de publication de la demande: 18.08.1993
(73) Titulaire: COMMISSARIAT A L'ENERGIE ATOMIQUE, 75015 Paris (FR)
(72) Inventeur: Boulain, Jean-Claude, F-91120 Palaiseau (FR); Ducancel, Frédéric, 12 rue de l'Yvette 91160 Longjumeau (FR); Gillet, Daniel, F-75020 Paris (FR); Menez, André, F-78470 Saint Remy les Chevreuses (FR)
(74) Mandataire: Orès, Bernard

(56) Documents cités:
- EP-A- 0 407 259
- BIO/TECHNOLOGY vol. 9, no. 12, Décembre 1991, NATURE AMERICA, INC., NEW YORK, US pages 1373 - 1377 L.J. GARRARD ET AL. 'Fab assembly and enrichment in a monovalent phage display system'
- PROC. NATL. ACAD. SCI. vol. 87, no. 6, 1990, NATL. ACAD. SCI.,WASHINGTON, DC, US; pages 2047 - 2051 J. CASADEI ET AL. 'Expression and secretion of aequorin as a chimeric antibody by means of a mammalian expression system'
- NATURE vol. 339, 1 Juin 1989, MACMILLAN JOURNALS LTD., LONDON, UK pages 394 - 397 V.K. CHAUDHARY ET AL. 'A recombinant immunotoxin consisting of two antibody variable domains fused to Pseudomonas exotoxin'
- BIO/TECHNOLOGY vol. 9, no. 6, Juin 1991, NATURE AMERICA, INC., NEW YORK, US pages 545 - 551 A. PLüCKTHUN 'Antibody engeneering: Advances from the use of Escherichia coli expression system'

## Description

La présente invention est relative à des protéines hybrides complexes comprenant les chaînes légères et les régions variables et certains domaines constants des chaînes lourdes des immunoglobulines (anticorps) et une autre protéine homo- ou hétérodimérique, à leur procédé de préparation, ainsi qu'à leurs applications comme agent de diagnostic, (notamment les anticorps recombinants bivalents et éventuellement colorimétriques obtenus à partir de ces protéines hybrides), comme agent à visée thérapeutique ou comme réactif utilisable en imagerie médicale.

Les anticorps monoclonaux ont été obtenus initialement à partir de la technique des hybridomes décrite par KOLHER et MILSTEIN en 1975 (Nature, 256, 495-497). Depuis, de nombreuses techniques ont été développées, qui permettent de manipuler les séquences nucléotidiques codant pour des immunoglobulines. Ainsi, il est devenu possible de choisir l'organisme qui les produit et de modifier leur structure afin de l'adapter à l'utilisation souhaitée.

La structure de base d'une immunoglobuline comprend deux chaînes polypeptidiques légères identiques et deux chaînes lourdes identiques reliées par des ponts disulfures. Les régions N-terminales des chaînes lourdes (H) et des chaînes légères (L) sont caractérisées par des séquences variables (V) appelées respectivement régions V_{H} et V_{L}. Le reste de la molécule a une structure relativement constante. La partie constante de la chaîne légère est appelée région C_{L} et la partie constante de la chaîne lourde comprend au moins trois régions différentes : C_{H}1, C_{H}2 et C_{H}3 (IgG, par exemple) ; ces régions globulaires, stabilisées par des ponts disulfures intracaténaires, sont également appelées "domaines". Chacun des deux sites de liaison à l'antigène est constitué par l'assemblage des domaines variables (V_{H} et V_{L}) et les fonctions effectrices (fixation du complément, liaison sur les monocytes et passage à travers le placenta) sont plutôt localisées au niveau du domaine C_{H}3 ; la région charnière est localisée entre C_{H}1 et C_{H}2. Il est possible par clivage avec différentes enzymes protéolytiques de réduire la taille d'une immunoglobuline, tout en conservant au moins un fragment de liaison fonctionnel à l'antigène. Ainsi, la pepsine libère le fragment F(ab')₂, bivalent, constitué de deux fragments Fab reliés par la région charnière. La papaïne clive la molécule d'IgG en libérant deux fragments Fab identiques, monovalents, qui se lient à l'antigène. Le plus petit fragment contenant un site de liaison complet à l'antigène est le fragment Fv, qui est constitué de l'association des domaines V_{H} et V_{L}. Cependant, l'isolement de ce fragment s'avère difficile. De plus, son affinité pour l'antigène et sa stabilité sont diminuées (J.S. HUSTON et al., 1988, P.N.A.S. 85, 5879-5883).

Les modifications au niveau de la production des anticorps monoclonaux, telles que décrites dans l'Art antérieur comprennent :
. Des techniques d'ingénierie génétique des anticorps, qui permettent d'avoir accès à l'information génétique responsable de la synthèse des immunoglobulines, de modifier cette information à façon et de la réintroduire modifiée ou non dans des cellules d'hybridomes obtenues selon le procédé décrit dans les travaux initiaux de KOHLER et MILSTEIN en 1975 (Nature, 256, 495-497), qui permettent d'obtenir des ensembles d'anticorps homogènes et de spécificité prédéfinie, utilisés dans les domaines thérapeutique et du diagnostic, ou des organismes divers, en particulier des microorganismes, qui produiront les protéines correspondantes.
. Les travaux initiaux rapportant la production chez *E. coli* de molécules apparentées aux immunoglobulines datent de 1984. Ils concernent la production intracytoplasmique d'une ou deux chaînes d'immunoglobuline (CABILLY et al., PNAS, 1984, 81, 3273-3277 ; BOSS et al., Nucl. Acids Res., 1984, 12, 3791-3806). La préparation d'un anticorps fonctionnel à partir de ces synthèses intracytoplasmiques nécessitaient des étapes de purification, solubilisation et renaturation *in vitro* lourdes et difficiles.
. Depuis, il est devenu possible de produire des fragments d'anticorps chez *E. coli* directement dans un état natif et fonctionnel. Seule la sécrétion séparée, mais simultanée des deux chaînes (ou d'un fragment simple chaîne) permet actuellement d'obtenir ce résultat. Ainsi des fragments Fab, Fv, scFV, dAb, Fc, mru, (voir nomenclature dans la revue de WINTER et MILSTEIN citée précédemment) ont été produits ; il est également possible d'exposer ces molécules à la surface de bactériophages (McCAFFERTY et al., 1990, Nature, 348, 552-554 ; CLACKSON et al., 1991, Nature, 352, 624-628).

Il faut cependant noter que, dans tous les cas, les molécules obtenues sont monovalentes, c'est-à-dire ne comportent qu'un site de liaison à l'antigène.
. La production d'anticorps ou de produits dérivés ont été également décrits dans de nombreux autres organismes, tels que des champignons, des levures, des cellules de mammifère, des cellules végétales ou des systèmes d'expression mixtes phage/cellule hôte comme les systèmes utilisant le baculovirus.

Les molécules dérivées des immunoglobulines produites par des bactéries ou des phages et décrites dans l'Art antérieur, présentent les avantages suivants : manipulations génétiques plus faciles à mettre en oeuvre et plus riches en potentialités (voir les systèmes de criblage développés chez les phages), et la production en masse de ces nouvelles protéines s'en trouve également simplifiée ; toutefois, ces molécules ont l'inconvénient majeur d'avoir une partie immunoglobuline monovalente, c'est-à-dire ne comportant qu'un seul site de liaison à l'antigène.

A partir de ces différents modes de production, il est possible de modifier la structure des anticorps et de produire des anticorps recombinants possédant de nouvelles propriétés : anticorps humanisés, bispécifiques, hybrides ; l'Art antérieur décrit notamment :
- des anticorps de rongeurs "humanisés", dans le but notamment de réduire la réponse immunitaire à ces anticorps, lors de leur administration chez l'Homme ; de tels anticorps sont notamment obtenus par liaison des régions variables d'immunoglobulines de rongeurs avec les régions constantes d'immunoglobulines humaines ; de tels anticorps, produits par des cellules d'hybridomes, ont une immunogénicité réduite (G. WINTER et C. MILSTEIN, Nature, 349, 293-299).
- des anticorps, dont chacun des deux sites de liaison aux antigènes a une spécificité différente (bispécificité de l'anticorps) ont été réalisés ; ils peuvent se lier simultanément à une cellule cible et à une toxine ou à une cellule T cytotoxique et entraîner la destruction de la cellule cible (J.L. PHELPS et al., J. Immunol., 1990, 145, 4, 1200-1204) ; de tels anticorps peuvent être produits, par exemple, par des hybridomes hybrides ou l'introduction de séquences codant pour des immunoglobulines de deux spécificités différentes dans des cellules de myélome (revue de T.A. WALDMANN, 1991, Science, 252, 1657-1662).
- l'introduction de nouvelles fonctions, par fusion de fragments d'immunoglobulines à d'autres protéines, peut également être réalisée. On peut citer par exemple NEUBERGER et al. (Nature, 1984, 312, 604-608) qui ont réalisé la fusion de gènes codant pour une chaîne lourde tronquée d'immunoglobuline avec celui d'une nucléase bactérienne ou de la protéine oncogène *myc,* et qui ont exprimé ces hybrides avec la chaîne légère correspondante dans une cellule de myélome. Selon le même principe, SCHNEE et al. (1987, PNAS, 84, 6904-6908) ont réalisé une protéine hybride bifonctionnelle constituée d'une partie immunoglobuline reconnaissant spécifiquement la fibrine et d'une région activateur tissulaire du plasminogène (t-PA), formant ainsi une protéine à action thrombolytique. CASADEI et al. (1990, PNAS, 87, 2047-2051) quant à eux ont construit un hybride anticorps-aéquorine. En choisissant *E. coli* comme cellule hôte, CHAUDHARY et al. (1989, Nature, 339, 394-397) ont réalisé des hybrides entre un fragment Fv simple chaîne (sFv) d'immunoglobuline et une exotoxine de *Pseudomonas*, exprimés de façon intracellulaire. De récents travaux (BRINKMANN et al., 1991, PNAS, 88, 8616-8620) montrent que l'une de ces constructions se révèle efficace dans le traitement de carcinomes humains étudiés chez la souris.

Dans ces différents documents, le gène de fusion obtenu génère une protéine de fusion bifonctionnelle, c'est-à-dire possédant des fonctions appartenant aux deux protéines qui la constituent (immunoglobuline et autre protéine). Bien que ces molécules hybrides bifonctionnelles aient élargi le domaine d'application des immunoglobulines monoclonales (traitement des cancers, thromboses, réactifs de diagnostic, etc...), il apparaît cependant des problèmes de structuration de ces protéines recombinantes. NEUBERGER et al., et CASADEI et al. indiquent, dans leurs publications, que des problèmes d'association des deux chaînes lourdes d'immunoglobuline apparaissent qui peuvent être dus à la présence, en place de la région Fc, de la seconde protéine (nucléase ou aéquorine). SCHNEE et al. précisent que la présence de l'activateur de plasminogène entraîne une diminution importante de l'expression de la protéine hybride dans l'hybridome. Enfin, les protéines hybrides obtenues chez *E. coli* par CHAUDHARY et al. sont localisées dans le cytoplasme et doivent être purifiées et renaturées. En outre, elles ne possèdent qu'un site de liaison à l'antigène constitué d'un fragment Fv simple chaîne dont l'affinité pour l'antigène peut être diminuée, avec comme référence un fragment Fab.

L'Art antérieur précité montre qu'il apparaît que la généralisation de la production d'une protéine hybride comprenant un anticorps bivalent, associé à une autre protéine, avec le maintien des fonctionnalités des deux protéines n'est pas évidente et que de nombreux facteurs ne sont ni maîtrisés (appariement des chaînes lourdes et légères notamment), ni généralisables.

En particulier, la production d'une protéine hybride par une bactérie, comprenant une partie immunoglobuline dite bivalente, c'est-à-dire possédant deux sites de liaison aux antigènes, bifonctionnelle (portant des fonctions des deux protéines fusionnées), et produite directement sous une forme structurée et active n'est pas résolue. La simple obtention directe d'un fragment d'anticorps bivalent fonctionnel (non fusionné à une autre protéine) à partir d'une bactérie n'est d'ailleurs pas non plus résolue, puisque seuls des fragments monovalents ont été obtenus jusqu'à présent.

La Demanderesse a résolu le problème de la généralisation de la production dans des microorganismes de telles protéines hybrides bifonctionnelles et bivalentes exportées et naturellement structurées, comportant un fragment d'immunoglobuline (sFv) dans sa Demande de Brevet européen 407 259.

Dans la présente Demande, le problème à résoudre concerne la production directe de protéines bifonctionnelles comportant une partie immunoglobuline de structure de type F(ab)₂ associée à une autre protéine fonctionnelle.

La présente invention s'est en conséquence donné pour but de pourvoir à de telles protéines hybrides comprenant un fragment d'immunoglobuline de structure de type F(ab)₂, qui répondent mieux aux besoins de la pratique que les protéines hybrides bifonctionnelles et formant un anticorps de l'Art antérieur, notamment en ce qu'elles présentent des sites de liaison aux antigènes plus proches des sites naturels que les fragments Fv, une plus grande spécificité et une plus grande affinité pour les antigènes, ce qui conduit notamment à une amélioration significative des tests immunologiques dans lesquels ces nouvelles protéines hybrides sont utilisées.

On entend, au sens de la présente invention, par :
- bifonctionnelle, une protéine hybride, comprenant un fragment d'immunoglobuline et une autre protéine, présentant des fonctions issues des deux protéines dont elle est constituée ;
- monovalente, une protéine hybride telle que définie ci-dessus, comportant un seul site de liaison à l'antigène ;
- bivalente, une protéine hybride bifonctionnelle telle que définie ci-dessus, comportant deux sites de liaison à un antigène ; dans le cas où les deux sites sont identiques, la protéine hybride est dite monospécifique ; dans le cas où les deux sites de liaison sont différents, la protéine hybride est dite bispécifique.

La présente invention a pour objet une protéine hybride du type comprenant un fragment d'immunoglobuline fusionné à une autre protéine P, caractérisée en ce qu'elle est constituée par une séquence hybride complexe comprenant :
. **une unité de base U constituée par :**
   - deux chaînes peptidiques différentes SU₁ et SU₂, SU₁ comprenant les régions variables et constantes d'une chaîne légère d'une immunoglobuline et SU₂ comprenant au moins les régions variables et le domaine C_{H}1 d'une chaîne lourde d'une immunoglobuline, lesquelles chaînes peptidiques SU₁ et SU₂ sont reliées entre elles par au moins un pont disulfure, lesdites chaînes peptidiques SU₁ et SU₂ comprenant en amont de leur extrémité N-terminale, le fragment d'une protéine P₁ correspondant au signal d'exportation ou de sécrétion de ladite protéine P₁, exportée ou sécrétée par une cellule hôte unicellulaire choisie dans le groupe constitué par les souches bactériennes ou fungiques (*E. coli, Bacillus, Streptomyces*), les levures, les cellules de mammifère, les cellules végétales, ou les systèmes d'expression mixtes phage/cellule hôte comme les systèmes utilisant le baculovirus, et
   - au moins un fragment fonctionnel d'une des deux sous-unités d'une protéine P dimérique comprenant le domaine nécessaire à la dimérisation de la protéine P, fusionnée par son extrémité N-terminale à l'une des chaînes peptidiques SU₁ ou SU₂,
. **laquelle unité de base U est associée à une autre unité de base U'**, identique ou différente de l'unité U, par l'intermédiaire des sous-unités de la protéine P ou un fragment de celles-ci.

Les sous-unités de la protéine P s'assemblent par dimérisation, pour former la protéine P fonctionnelle. On entend donc, au sens de la présente invention, par fragment fonctionnel, un fragment de la protéine P susceptible de présenter la propriété recherchée de ladite protéine P, lequel fragment est généralement sous forme dimérique.

La protéine hybride conforme à l'invention, dite protéine hybride complexe, forme, lorsqu'elle est exportée à partir d'une cellule hôte unicellulaire, telle que les souches bactériennes ou fungiques *(E. coli*, *Bacillus*, *Streptomyces*), des levures, des cellules de mammifère, des cellules végétales, ou des systèmes d'expression mixtes phage/cellule hôte comme les systèmes utilisant le baculovirus, dans laquelle elle est exprimée, un dimère fonctionnel possédant :
- au moins deux sites de liaison à un antigène, identiques ou différents et portés chacun par l'une des unités de base (U, U'...) de ladite protéine hybride,
- et une fonction biologique liée aux proprités intrinsèques de la protéine P homo ou hétérodimérique additionnelle.

Ces nouvelles protéines répondent ainsi particulièrement aux besoins de la pratique, par rapport aux protéines hybrides bifonctionnelles de l'Art antérieur produites dans des cellules de myélome et dont l'association des deux fragments Fab' en un fragment F(ab')2 par formation d'un ou plusieurs ponts disulfure peut être altérée par la présence de l'autre protéine, en ce sens que l'association des deux fragments Fab, dans la protéine hybride complexe selon l'invention, est assurée par l'interaction naturelle qui se crée entre les deux sous-unités de la protéine dimérique fusionnée à une chaîne ou un fragment de chaîne de l'immunoglobuline. Elles présentent, de plus, l'avantage de pouvoir être, simultanément, bifonctionnelles et bispécifiques.

Selon un mode de réalisation avantageux de ladite protéine hybride complexe, la protéine P1 est différente de la protéine P.

Selon un autre mode de réalisation avantageux de ladite protéine hybride complexe, la protéine P1 est identique à la protéine P.

Selon un autre mode de réalisation avantageux de ladite protéine hybride complexe, la protéine P dimérique est choisie dans le groupe qui comprend les protéines homodimériques formées de 2 sous-unités identiques et les protéines hétérodimériques formées de 2 sous-unités différentes.

Parmi les protéines P particulièrement intéressantes, on peut citer les toxines homodimériques (toxine tétanique, toxine botulique, par exemple), les enzymes homodimériques, telles que la phosphatase et plus particulièrement la phosphatase alcaline de différentes origines, naturelles ou modifiées génétiquement, afin d'améliorer leur performance enzymatique (XUXU et al., Biochem., 1991, 30, 7789-7796) ; on peut citer comme protéine hétérodimérique, notamment une phosphatase alcaline dont les deux sous-unités sont modifiées de façon complémentaire au niveau de leur interface, par exemple en inversant un pont salin ou bien des toxines hétérodimériques ou des complexes ligand/fraction soluble de récepteur.

Conformément à l'invention, ladite protéine hybride complexe comprend deux unités de base U et U' identiques ou différentes, constituées chacune :
- par une chaîne peptidique SU₁ comprenant les régions variables et constantes d'une chaîne légère d'une immunoglobuline appropriée (V_{L} + C_{L}), par une chaîne peptidique SU₂ comprenant les régions variables et le domaine C_{H}1 d'une chaîne lourde de la même immunoglobuline (V_{H} + C_{H}1), lesquelles chaînes SU₁ et SU₂ sont reliées entre elles par au moins un pont disulfure,
- et par un fragment fonctionnel d'une phosphatase alcaline (protéine P) comprenant le domaine nécessaire à la dimérisation de la protéine P, exportée ou excrétée par une cellule hôte appropriée, fusionnée par son extrêmité, N-terminale à l'une des chaînes SU₁ ou SU₂, lesquelles unités de base U et U' sont réunies par l'assemblage des deux sous-unités de ladite phosphatase alcaline dimérique. On obtient alors un tétramère fonctionnel, qui possède, de manière surprenante, deux sites de liaison fonctionnels à l'antigène correspondant à ladite immunoglobuline et une fonction biologique liée aux propriétés intrinsèques de la protéine dimérique additionnelle.

Dans le cas où la protéine additionnelle (P) est une enzyme et plus particulièrement une phosphatase alcaline, ladite protéine hybride complexe est un "anticorps colorimétrique" directement utilisable, dans le domaine diagnostic (réactif de diagnostic, réactif en imagerie médicale).

La présente invention a également pour objet une unité de transcription T1 d'une protéine hybride complexe conforme à l'invention, caractérisée en ce qu'elle comprend au moins successivement :
. un promoteur approprié,
. un premier site de fixation aux ribosomes (séquence de Shine-Dalgarno),
. une première séquence A comprenant une séquence signal d'une protéine P1 exportée ou excrétée par une cellule hôte et susceptible de permettre l'exportation des immunoglobulines associées,
. une séquence C d'acide nucléique constituée par une séquence choisie dans le groupe constitué soit par une séquence codant au moins pour la région variable et le domaine C_{H}1 de la chaîne lourde d'une immunoglobuline, soit par une séquence codant pour la chaîne légère d'une immunoglobuline,
. une séquence D codant au moins pour un fragment fonctionnel comprenant le domaine nécessaire à la dimérisation de la protéine P, d'une sous-unité de la séquence mature d'une protéine P dimérique,
. au moins un codon stop,
. un deuxième site de fixation aux ribosomes (séquence de Shine-Dalgarno),
. une deuxième séquence A telle que définie ci-dessus,
. une séquence E d'acide nucléique codant pour la séquence de la chaîne non codée par la séquence C ci-dessus,
. un ou plusieurs codon stop,
. et un terminateur de transcription.

Une telle construction permet, de manière surprenante, lorsqu'elle est convenablement exprimée, l'obtention d'une protéine hybride complexe, telle que définie ci-dessus, directement utilisable notamment comme réactif de diagnostic.

Selon un mode de réalisation avantageux de ladite unité de transcription T1, elle comprend en outre, entre la première séquence A et la séquence C et entre la deuxième séquence A et la séquence E ci-dessus, une séquence B d'acide nucléique codant pour un fragment NH2-terminal de la protéine P₁ mature.

Selon une disposition avantageuse de ce mode de mise en oeuvre, P est choisie dans le groupe qui comprend les protéines homodimériques formées de 2 sous-unités identiques et les protéines hétérodimériques formées de 2 sous-unités différentes.

Selon une autre disposition avantageuse de ce mode de réalisation P₁ et P sont identiques.

Conformément à cette dernière disposition, ladite unité de transcription T1 est constituée par :
. un promoteur approprié,
. un premier site de fixation aux ribosomes (séquence de Shine-Dalgarno),
. une première séquence A comprenant une séquence signal du gène de structure d'une protéine homo- ou hétérodimérique exportée ou sécrétée par une cellule hôte,
. une séquence B comprenant une séquence d'acide nucléique codant pour un fragment NH₂-terminal de la protéine homo- ou hétérodimérique mature,
. une séquence C constituée par une séquence choisie dans le groupe constitué, soit par une séquence d'acide nucléique codant pour la région variable et le domaine C_{H}1 de la chaîne lourde d'une immunoglobuline, soit par une séquence codant pour la chaîne légère d'une immunoglobuline,
. une séquence D constituée par un fragment codant pour au moins un fragment fonctionnel comprenant le domaine nécessaire à la dimérisation de la protéine P, de la séquence mature de ladite protéine homo- ou hétéro-dimérique,
. au moins un codon stop,
. un deuxième site de fixation aux ribosomes,
. une deuxième séquence A telle que définie ci-dessus,
. une séquence B comprenant une séquence d'acide nucléique codant pour un fragment NH₂-terminal de la protéine homo- ou hétérodimérique mature,
. une séquence E constituée par une séquence d'acide nucléique codant pour la chaîne de ladite immunoglobuline, non codée par la séquence C ci-dessus,
. au moins un codon stop,
. et un terminateur de transcription,
l'ensemble de ces fragments étant en phase de lecture et codant pour la protéine hybride complexe telle que définie ci-dessus, présentant à la fois les propriétés de l'enzyme et certaines propriétés des fragments Fab des immunoglobulines (capacité de reconnaissance de l'antigène correspondant).

Lorsque P est plus spécifiquement une phosphatase alcaline, ladite unité de transcription T1 est constituée :
- **soit par :**
   . un promoteur approprié,
   . un premier site de fixation aux ribosomes (séquence de Shine-Dalgarno),
   . une première séquence A comprenant une séquence signal d'une protéine P₁, susceptible de permettre l'exportation des immunoglobulines associées,
   . une séquence C d'acide nucléique choisie dans le groupe constitué soit par une séquence codant pour la région variable et le domaine C_{H}1 de la chaîne lourde d'une immunoglobuline, soit par une séquence codant pour la chaîne légère d'une immunoglobuline,
   . un fragment D codant pour la phosphatase alcaline,
   . au moins un codon stop,
   . un deuxième site de fixation aux ribosomes,
   . une deuxième séquence A telle que définie ci-dessus,
   . une séquence E d'acide nucléique codant pour la chaîne non codée par la séquence C ci-dessus de ladite immunoglobuline,
   . au moins un codon stop,
   . et un terminateur de transcription,
   laquelle unité de transcription comprend en outre, notamment pour permettre l'insertion des fragments codant pour les immunoglobulines et pour la mise en phase de lecture de l'ensemble de ces fragments, en amont et/ou en aval de la séquence C et en amont et/ou en aval de la séquence E, un fragment d'acide nucléique approprié, laquelle unité de transcription, en phase de lecture, code pour une protéine hybride telle que définie ci-dessus ;
- **soit par** (avec P₁ identique à P) :
   . un promoteur approprié,
   . un premier site de fixation aux ribosomes (séquence de Shine-Dalgarno),
   . une première séquence A comprenant la séquence signal du gène de structure de la phosphatase alcaline,
   . un fragment B codant pour les 6 aminoacides N-terminaux de la phosphatase alcaline mature,
   . une séquence C constituée par une séquence d'acide nucléique choisie dans le groupe constitué soit par une séquence codant pour la région variable et le domaine C_{H}1 de la chaîne lourde d'une immunoglobuline, soit par une séquence codant pour la chaîne légère d'une immunoglobuline,
   . une séquence D constituée par un fragment codant pour les 444 aminoacides restants C-terminaux de la séquence mature de ladite enzyme dimérique,
   . au moins un codon stop,
   . un deuxième site de fixation aux ribosomes,
   . une deuxième séquence A telle que définie ci-dessus,
   . un fragment B codant pour les 6 aminoacides N-terminaux de la phosphatase alcaline mature,
   . une séquence E d'acide nucléique telle que définie ci-dessus codant pour la chaîne non codée par la séquence C ci-dessus de ladite immunoglobuline,
   . au moins un codon stop,
   . et un terminateur de transcription.

Une unité de transcription T1 conforme à l'invention, dans laquelle la séquence C est insérée après le premier triplet codant pour le 6ème aminoacide de la phosphatase alcaline mature et dans laquelle la séquence E est insérée après le deuxième triplet codant pour le 6ème aminoacide de la phosphatase alcaline, est notamment préparée grâce à l'insertion d'au moins un site de restriction unique dans la séquence codant pour la phosphatase alcaline, en chaque point d'insertion défini ci-dessus. Le site de restriction au niveau du premier triplet codant pour le 6ème aminoacide de la phosphatase alcaline, déphase le cadre de lecture du gène de la phoA et empêche l'expression de la phosphatase alcaline, alors que l'insertion de la séquence C au niveau dudit site permet, dans les conditions de l'invention, le rephasage du gène de la phosphatase alcaline et l'expression de cette dernière sous sa forme dimérique.

L'insertion d'au moins un site de restriction au niveau du second triplet codant pour le 6ème acide aminé et l'introduction de la séquence E n'influencent pas l'activité phosphatase.

Selon une modalité préférée de cette unité de transcription T1, elle comprend une première séquence A signal du gène de structure de la phosphatase alcaline, une première séquence B codant pour les 6 aminoacides N-terminaux de la phosphatase alcaline, un site de restriction Bgl II, une séquence C, choisie dans le groupe constitué soit par une séquence codant pour la région variable et le domaine C_{H}1 d'une chaîne lourde d'une immunoglobuline, soit par la séquence codant pour la chaîne légère d'une immunoglobuline, un site de restriction Sal I, un fragment D codant pour les 444 aminoacides restants C-terminaux de la phosphatase alcaline, une deuxième séquence A, une deuxième séquence B, un site de restriction Sac I, une séquence E codant pour la séquence non codée par la séquence C, un site de restriction XbaI, au moins un codon stop et un terminateur de transcription.

Une telle unité de transcription T1 est dite chargée, car elle est directement apte à coder pour une protéine hybride complexe conforme à l'invention, lorsqu'elle est insérée dans un vecteur approprié, comprenant notamment une origine de réplication.

La présente invention a également pour objet une unité de transcription T2, caractérisée en ce qu'elle comprend :
. un promoteur approprié,
. un site de fixation aux ribosomes (séquence de Shine-Dalgarno),
. une première séquence A signal d'un gène de structure d'une protéine P₁, une séquence D apte à coder pour un fragment fonctionnel de la séquence mature d'une protéine P dimérique et un ou plusieurs sites de restriction uniques, situés à la jonction entre la première séquence A et la séquence D et apte à recevoir une séquence C telle que définie ci-dessus,
. au moins un codon stop,
. un site de fixation aux ribosomes (séquence de Shine-Dalgarno),
. une deuxième séquence A telle que définie ci-dessus et un ou plusieurs sites de restriction uniques, situés en aval de ladite séquence A et aptes à recevoir une séquence E telle que définie ci-dessus,
. au moins un codon stop,
. et un terminateur de transcription.

Selon un mode de réalisation avantageux de l'unité de transcription T2, elle comprend :
. un promoteur approprié,
. un site de fixation aux ribosomes (séquence de Shine-Dalgarno),
. une première séquence A signal d'un gène de structure d'une protéine P₁,
. une première séquence B codant pour un fragment NH₂-terminal de ladite protéine P₁ mature,
. une séquence D apte à coder pour une protéine P mature ou un fragment de celle-ci et un ou plusieurs sites de restriction uniques, situés à la jonction entre le fragment NH₂-terminal de séquence codant pour la protéine P₁ et la séquence codant pour la protéine P et aptes à recevoir une séquence C telle que définie ci-dessus,
. au moins un codon stop,
. un site de fixation aux ribosomes (séquence de Shine-Dalgarno),
. une deuxième séquence A telle que définie ci-dessus,
. une deuxième séquence B telle que définie ci-dessus et un ou plusieurs sites de restriction uniques, situés en aval de ladite séquence A et aptes à recevoir une séquence E telle que définie ci-dessus,
. au moins un codon stop,
. et un terminateur de transcription.

Selon une disposition avantageuse de ce mode de réalisation, P₁ et P sont identiques (protéine homo- ou hétérodimérique).

Conformément à l'invention, le promoteur est choisi dans le groupe qui comprend le promoteur du gène phoA et tout autre promoteur.

La présente invention a également pour objet des vecteurs d'expression et/ou de clonage d'une protéine hybride complexe conforme à l'invention, caractérisés en ce que chaque vecteur inclut une unité de transcription T1 conforme à l'invention, insérée dans une structure génétique appropriée, notamment choisie dans le groupe qui comprend des plasmides, des phages, des cosmides ou des chromosomes appropriés.

Selon un mode de réalisation avantageux dudit vecteur, la structure génétique est un plasmide, et l'unité de transcription T1 comprend un gène ou un fragment de gène codant pour une protéine P₁, identique à la protéine P, laquelle protéine P est une enzyme dimérique.

La présente invention a également pour objet des vecteurs potentiels d'expression et/ou de clonage d'une protéine hybride complexe conforme à l'invention, caractérisés en ce que chaque vecteur inclut une unité de transcription T2 conforme à l'invention, insérée dans une structure génétique appropriée, notamment choisie dans le groupe qui comprend des plasmides, des phages, des cosmides ou des chromosomes appropriés.

Un tel vecteur est dénommé pLIP2.

Lorsque ladite structure est un plasmide, ce dernier est dit non chargé ; lorsque les séquences C et E sont insérées au niveau des sites de restriction uniques tels que définis ci-dessus, de tels vecteurs contiennent des séquences hybrides conformes à l'invention et expriment directement la protéine hybride, notamment les anticorps colorimétriques tels que définis ci-dessus.

De tels vecteurs peuvent notamment être préparés à partir du vecteur non chargé dénommé pLIP1 décrit dans la Demande de Brevet européen 407 259, au nom de la Demanderesse.

La présente invention a également pour objet un microorganisme obtenu par transformation génétique, caractérisé en ce qu'il est obtenu par modification appropriée d'une souche d'*E. coli* par un vecteur conforme à l'invention et notamment par transformation, lorsque ledit vecteur est un plasmide.

Selon un mode de réalisation de l'invention, ledit microorganisme est avantageusement une souche CC118 d'*E. coli* transformée par un vecteur non chargé, comprenant une unité de transcription T2, tel que définie ci-dessus.

Selon une disposition avantageuse de ce mode de réalisation, ledit microorganisme a été déposé en date du 31 janvier 1992, sous le numéro I-1168 auprès de la Collection Nationale des Cultures de Microorganismes tenue par l'Institut Pasteur.

D'autres cellules hôtes peuvent également servir à la production de molécules hybrides complexes conformes à l'invention ; on peut notamment citer, et ce de manière non limitative, les souches bactériennes ou fungiques (telles que *E. coli, Bacillus*, *Streptomyces*), les levures, les cellules de mammifère, les cellules végétales, ou les systèmes d'expression mixtes phage/cellule hôte comme les systèmes utilisant les baculovirus.

La protéine hybride complexe, conforme à l'invention présente les avantages des protéines hybrides telles que définies dans la demande de Brevet 407 259 (stabilité, utilisation directe, propriétés fonctionnelles des deux fragments protéiques contenus) et, de manière plus spécifique de telles protéines hybrides complexes possèdent deux fragments Fab associés identiques ou différents, selon le cas, en raison de la dimérisation de la protéine P.

Dans le cas où la protéine P est la phosphatase alcaline, l'anticorps colorimétrique produit chez la bactérie *E. coli* constitue un nouvel outil de diagnostic facile à produire ; son caractère bivalent (deux sites de fixation à l'antigène) lui confère une affinité apparente pour l'antigène reconnu plus importante que celle d'un fragment monovalent. Il en résultera une sensibilité de détection significativement plus importante.

Comparée au composé homologue réalisé par voie chimique, la protéine hybride synthétisée par la bactérie est beaucoup plus simple à produire ; en effet, pour réaliser un traceur performant par voie chimique, l'Homme de l'Art purifie l'anticorps monoclonal, le clive par des méthodes enzymatiques afin d'éliminer le fragment Fc dont la présence entraîne, en particulier, des interactions non spécifiques, purifie le fragment F(ab)'₂ qui est ensuite couplé à l'enzyme choisie par des méthodes de couplage chimique, puis le complexe est enfin purifié.

Dans la présente invention, la bactérie synthétise, exporte et assemble un complexe F(ab)₂/phosphatase alcaline bifonctionnel, utilisable après une étape d'extraction simple. En outre, la reproductibilité d'une préparation à une autre est assurée, ce qui n'est généralement pas le cas dans les préparations réalisées par des méthodes chimiques.

La présente invention a également pour objet un réactif de diagnostic, caractérisé en ce qu'il est constitué par une protéine hybride complexe conforme à l'invention.

Un tel réactif trouve notamment application dans des dosages immunoenzymatiques, dans la détection de récepteurs ou en tant que marqueur immunochimique.

La présente invention a également pour objet un procédé de dosage immunoenzymatique de protéines, caractérisé en ce qu'il consiste :
- à mettre en contact un échantillon biologique convenable avec un réactif de diagnostic conforme à l'invention et
- à détecter ledit réactif de diagnostic sous forme de complexe et/ou sous forme libre par une réaction colorimétrique appropriée.

La présente invention a, de plus, pour objet une trousse prête à l'emploi pour la mise en oeuvre dudit dosage immunoenzymatique, caractérisée ne ce qu'elle comprend outre les quantités appropriées de tampons et réactifs utiles pour la mise en oeuvre du dosage, des quantités appropriées du réactif conforme à l'invention.

Le réactif conforme à l'invention présente l'avantage d'être directement utilisable, alors qu'habituellement, dans le cadre d'un dosage EIA ou ELISA, l'antigène ou l'anticorps est lié de façon covalente à une enzyme, le couplage étant réalisé par voie chimique ; pour être efficace un tel couplage doit être réalisé avec deux réactifs hautement purifiés.

La présente invention a également pour objet un agent à visée thérapeutique, caractérisé en ce qu'il est constitué par une protéine hybride complexe conforme à l'invention.

Selon un mode de réalisation avantageux dudit agent, la protéine P est une substance cytotoxique, et les fragments d'immunoglobuline associés dans les unités de base sont d'origine humaine et présentent une spécificité vis-à-vis d'une cellule cible, préalablement sélectionnée.

La présente invention a en outre pour objet un procédé de production d'anticorps bivalents et bispécifiques, caractérisé en ce que :
- on produit, dans un vecteur conforme à l'invention, deux unités de transcription T1 dont les parties codant pour les domaines d'immunoglobulines sont différentes, lesquelles unités sont sous le contrôle du même promoteur et du même terminateur de transcription, puis, si nécessaire,
- on sélectionne les hybrides produits pour lesquels l'unité de base U est différente de l'unité de base U', notamment par chromatographie d'affinité.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

### EXEMPLE 1 : Anticorps colorimétriques bivalents monospécifiques produits chez un microorganisme.

### 1) Construction d'une unité dicistronique destinée à coexprimer deux fragments d'immunoglobuline dont un est fusionné à la phosphatase alcaline :

a. Introduction de deux sites de restriction dans le site SmaI du vecteur pLIP1 (réf. Demande de Brevet européen 407 259).
   Le vecteur pLIP1 est un plasmide portant le gène codant pour la phosphatase alcaline d'*E. coli* sous contrôle de son promoteur naturel. Un site de restriction SmaI unique inséré entre les codons +6 et +7 de la phosphatase alcaline mature déphase le cadre de lecture du gène phoA et permet l'insertion de séquences nucléotidiques diverses. Une cassette obtenue par synthèse de deux oligonucléotides complémentaires est insérée dans le site SmaI. Elle contient deux sites de restriction Bgl II et Sal I uniques et conserve le déphasage du cadre de lecture du gène de la phosphatase alcaline tel qu'il existe dans le vecteur pLIP1, ceci afin de permettre la sélection ultérieure des clones ayant inséré dans ces sites le fragment adéquat, en l'occurrence une séquence nucléotidique codant pour les domaines V_{H} et C_{H}1 d'une immunoglobuline.
b. Introduction d'une cassette synthétique entre le codon de terminaison du gène de la phosphatase alcaline et le signal de terminaison de transcription du même gène. Elle comprend successivement (unité de transcription T1) :
   - une partie 5' non codante du gène phoA comprennent une séquence de fixation aux ribosomes (séquence de Shine-Dalgarno),
   - la séquence codant pour le peptide signal et les six premiers acides aminés de la phosphatase alcaline,
   - deux sites de restriction uniques, Sac I et Xba I,
   - deux codons de terminaison.

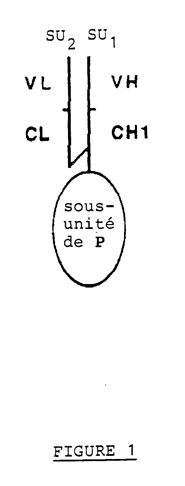

Cette portion du vecteur est conçue pour recevoir la chaîne V_{L} + C_{L} d'immunoglobuline, être cotranscrite, sous contrôle du promoteur phoA, avec le gène spécifiant l'hybride V_{H} + C_{H}1/phoA et traduite en protéine, telle que représentée à la figure 1, qui illustre une unité de base U, qui comprend une chaîne d'immunoglobuline SU₁, fusionnée à l'extrémité N-terminale d'une sous-unité de la protéine homo- ou hétéro-dimérique P. L'autre chaîne de l'immunoglobuline (SU₂) est associée à la protéine hybride par le biais du pont disulfure reliant les deux chaînes de l'immunoglobuline.

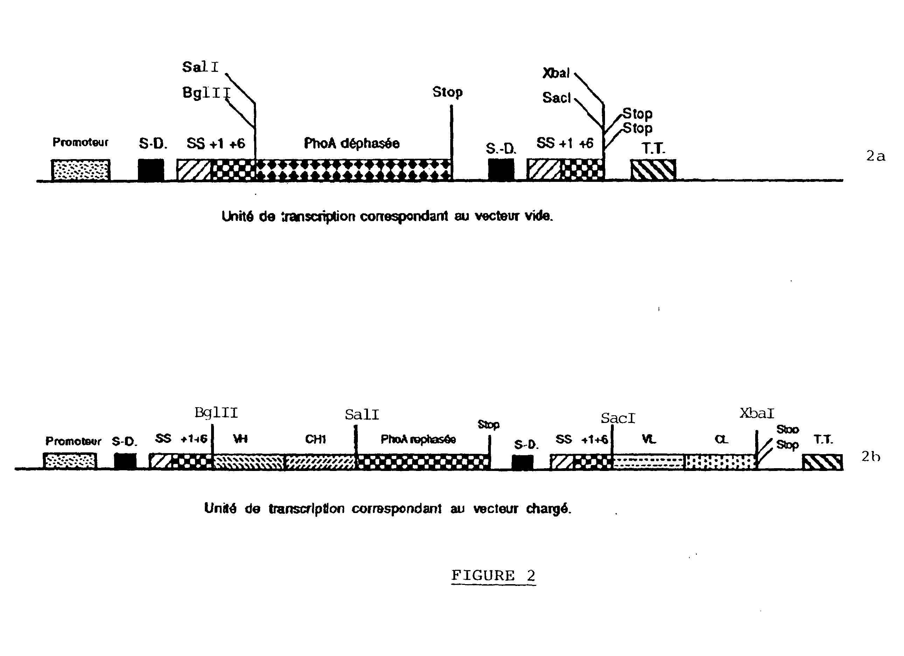

Le gène obtenu est représenté à la figure 2.

La figure 2a représente l'unité de transcription T2 (pLIP2 vide) (sans fragments d'immunoglobuline) et comprend successivement, à partir du promoteur, la séquence de Shine-Dalgarno (S.D.), la séquence signal de la phosphatase alcaline (SS), les résidus +1 +6 de la phosphatase mature (+1 +6), un site de restriction BglII, un site de restriction SalI, le gène déphasé codant pour la phosphatase alcaline (444 aminoacides) (PhoA déphasée), un codon stop, une séquence de Shine-Dalgarno (S.D.), une séquence signal de la phosphatase (SS), les résidus +1 +6 de la phosphatase mature (+1 +6), un site de restriction SacI, un site de restriction XbaI, deux codons stop et un terminateur de transcription (T.T.).

La figure 2b représente l'unité de transcription T1 (pLIP2 chargé), comprenant les fragments d'immunoglobulines : le domaine variable et le premier domaine constant de la chaîne lourde de l'immunoglobuline (V_{H} + C_{H}1), entre les sites de restrictions BglII et SalI et les domaines variable et constant de la chaîne légère de l'immunoglobuline, entre les sites de restrictions SacI et XbaI.

A partir du promoteur, l'ARN polymérase synthétise un transcrit unique qui s'arrête au niveau du terminateur de transcription (T.T.). La présence de deux sites de fixation des ribosomes (S.D.) permet, lors de la phase de traduction, la synthèse de deux protéines distinctes. La première est constituée du domaine variable et du premier domaine constant de la chaîne lourde de l'IgG, liés à l'extrémité N-terminale de la phosphatase alcaline. La seconde est constituée uniquement de la chaîne légère de l'IgG.

### 2) Préparation de fragments d'ADN codant pour une immunoglobuline.

L'anticorps monoclonal considéré est un anticorps spécifique des neurotoxines courtes de serpent, isolé et caractérisé, le Mα2-3 (IgG2aκ) (TREMEAU et al., 1986, FEBS Lett., 208, 236-240).

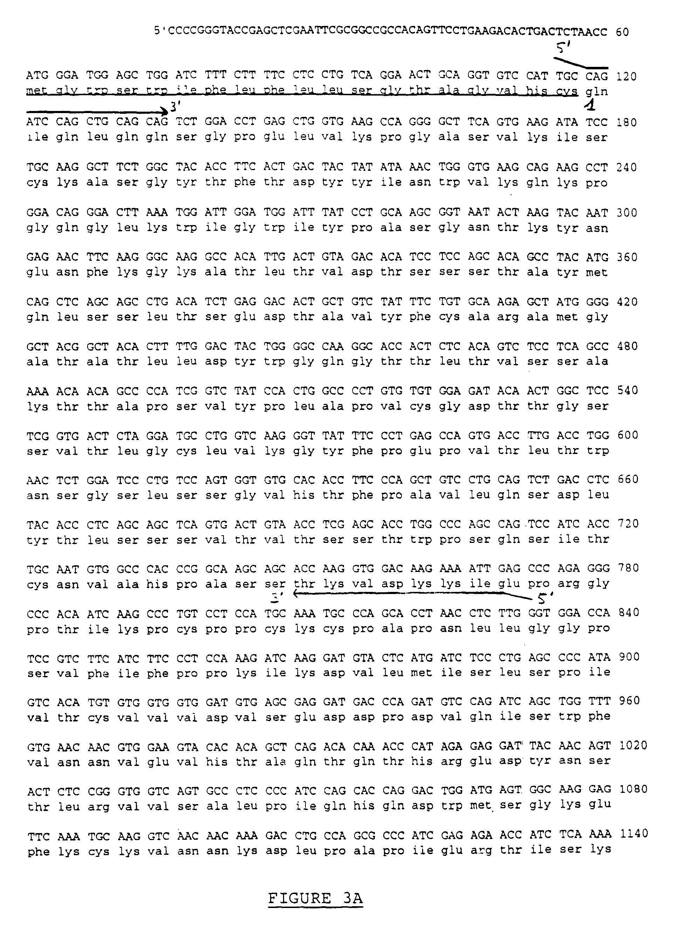

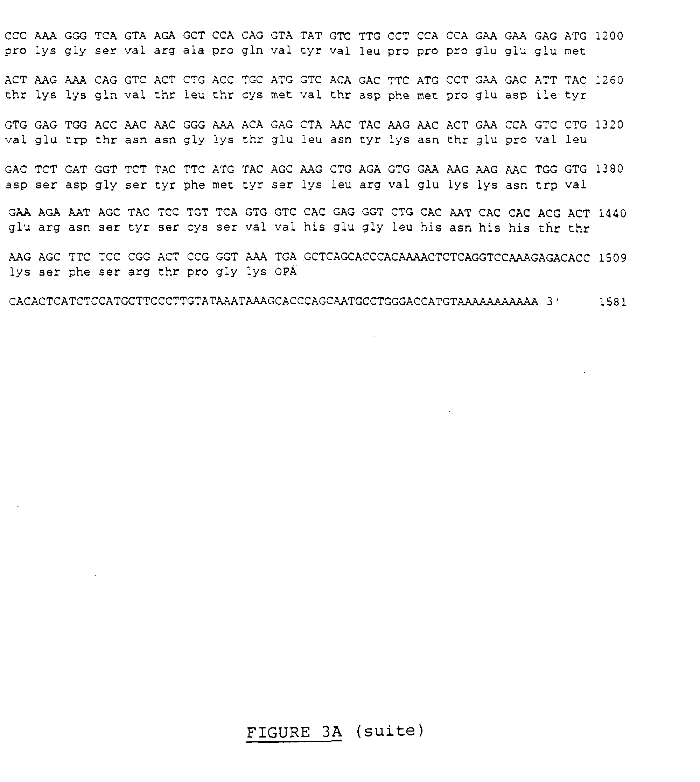

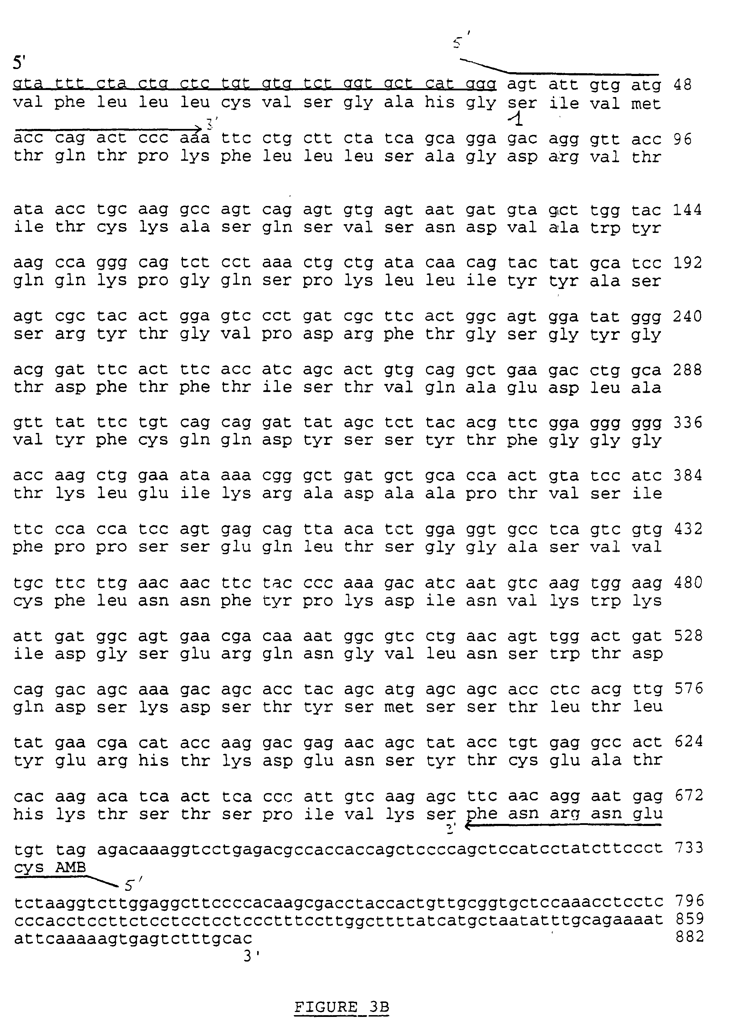

La figure 3A représente la séquence nucléotidique et la séquence protéique déduite, de la chaîne lourde de l'anticorps Mα2-3.

La figure 3B représente la séquence nucléotidique et la séquence protéique déduite, de la chaîne légère de l'anticorps Mα2-3.

La position des amorces nucléotidiques utilisées pour amplifier spécifiquement les fragments à insérer dans le vecteur pLIP2 et introduire les sites de restriction adéquats est indiquée par des flèches. la séquence de ces amorces est la suivante :
- Amorces chaîne lourde : 
5' GGGGGAGATCTCAGATCCAGCTGCAGCAGTCT 3'
5' GGGGGAGTCGACCAATTTTCTTGTCCACCTTGGT 3'
- Amorces chaîne légère :

5' GGAGCTCAGTATTGTGATGACCCAGACTCCCAA 3'
5' GTCTAGATTAACACTCATTTCTGTTGAA 3'.

L'ARN messager est extrait des cellules hybridomes sécrétant l'anticorps Mα2-3. La réverse transcriptase permet d'obtenir un double brin ARN/ADN selon les techniques classiques. Le brin matrice d'ARN est ensuite détruit et le second brin d'ADN est synthétisé grâce à l'ADN polymérase. Puis, les séquences d'ADN de la chaîne légère (V_{L} + C_{L}) et d'un fragment de la chaîne lourde (V_{H} + C_{H}1) de l'immunoglobuline sont obtenues par amplification spécifique des séquences correspondantes selon la technique de réaction de polymérisation en chaîne (PCR). Les amorces oligonucléotidiques utilisées pour cette amplification sont complémentaires des extrémités des fragments à amplifier et possèdent, à leur extrémité 5', les séquences des sites de restriction nécessaires à leur introduction dans le vecteur décrit dans l'exemple 1.1).

### 3) Introduction des fragments d'ADN de l'immunoglobuline dans le vecteur d'expression.

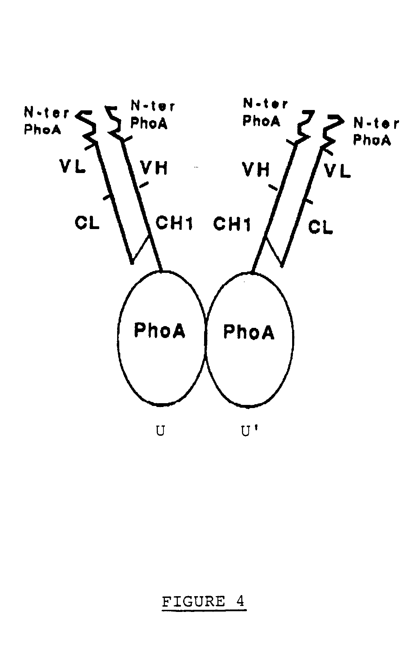

Le fragment V_{H} + C_{H}1 de la chaîne lourde est introduit au niveau des sites Bgl II et Sal I du vecteur (fusion au gène phoA). On dispose d'un crible colorimétrique pour cette insertion puisqu'elle permet le rephasage du cadre de lecture de la phosphatase alcaline qui suit l'insert. Ce crible témoigne également du fait que la phosphatase alcaline fusionnée à la chaîne d'immunoglobuline conserve tout ou partie de ses propriétés enzymatiques, notamment sa capacité à être exportée et à s'associer en un dimère fonctionnel. La chaîne légère (V_{L} + C_{L}) est introduite au niveau des sites Sac I et Xba I du vecteur. Sa présence est vérifiée par des études de restriction. L'ensemble de la construction est ensuite séquencé, afin de contrôler l'ensemble de la partie codante. La protéine hybride attendue, produit de traduction de cette construction est représentée à la figure 4 et comprend deux unités de base U et U' identiques ; il s'agit d'une protéine hybride complexe phosphatase alcaline/F(ab)₂.

### 4) Caractérisation et propriétés de la protéine recombinante obtenue.

En premier lieu, les clones bactériens obtenus hydrolysent, dans des conditions appropriées, le substrat X-P sur boîte de Pétri, lorsque le vecteur qu'ils contiennent comporte soit le fragment chaîne lourde seul, soit le fragment chaîne lourde et le fragment chaîne légère. Le vecteur vide ou chargé uniquement de la chaîne légère ne confère pas aux bactéries transformées par lui la capacité d'hydrolyser le substrat. Des résultats identiques sont obtenus en solution avec le substrat pNPP. Des chaînes polypeptidiques possédant un domaine phosphatase alcaline traversent donc la membrane cytoplasmique et s'associent en un dimère enzymatiquement actif.

Les immunoprécipitations réalisées avec des anticorps anti-phosphatase alcaline ou anti-immunoglobulines de souris montrent effectivement l'existence d'une protéine hybride entre enzyme et fragment V_{H} + C_{H}1 de l'immunoglobuline.

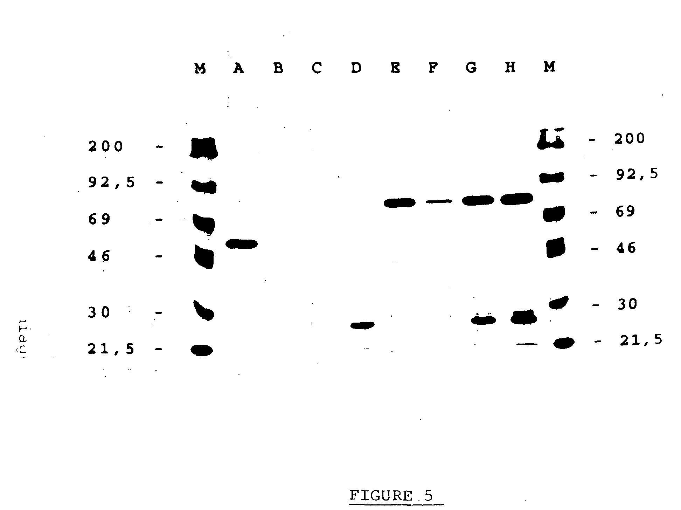

La figure 5 illustre les immunoprécipitations réalisées avec un anticorps monoclonal anti-phosphatase [VIAP] (puits A, C, E et G) ou avec un sérum polyclonal anti-chaînes lourdes (H) et légères (L) [anti-(H+L)] (puits B, D, F et H), à partir d'un extrait périplasmique bactérien. Les bactéries ont été transformées avec soit :
i) le plasmide initial ayant servi à construire les vecteurs pLIP1 et pLIP2 et contenant le gène natif de la phosphatase alcaline (puits A et B) ;
ii) le plasmide pLIP2 contenant la séquence codant pour la chaîne légère seule (puits C et D) ;
iii) le plasmide pLIP2 contenant le gène hybride V_{H}-C_{H}1-PhoA seul (puits E et F) ;
iv) le plasmide pLIP2 contenant l'opéron dicistronique totalement chargé (voir figure 2b), dans lequel les gènes V_{H}-C_{H}1-PhoA et V_{L}-C_{L} sont présents en même temps (puits G et H).

Dans une expérience contrôle, on a vérifié que les deux solutions d'anticorps utilisées ne précipitent aucune protéine du périplasme de bactéries transformées avec le vecteur vide. La présence (puits A) ainsi que l'absence (puits B) d'une bande ayant le poids moléculaire de la PhoA seule, caractérise la sélectivité des deux solutions d'anticorps utilisés. Les deux constructions intermédiaires, chaîne légère seule et hybride V_{H}-C_{H}1-PhoA sont respectivement caractérisées dans les puits C, D et E, F.

La chaîne légère est seulement (bande à 24 kD) immunoprécipitée par le sérum anti-(H+L), alors que l'hybride V_{H}-C_{H}1-PhoA (bande à 72 kD) est lui révélé à la fois par VIAP et le sérum anti-(H+L). Ces résultats démontrent sans ambiguïté, que l'opéron di-cistronique, décrit à la figure 2, est fonctionnel. Enfin, les bactéries transformées avec le plasmide chargé pLIP2/Fab, produisent dans leur périplasme deux protéines ayant les poids moléculaires attendus pour la chaîne légère et la chaîne lourde hybride. Ces deux composés sont indifféremment immunoprécipités par VIAP et le sérum anti-(H+L).

Des expériences de chasse ont été réalisées en bloquant le promoteur phoA par addition de phosphate. Après une heure de chasse, aucune diminution des bandes immunoprécipitées n'est enregistrée et il n'apparaît pas de bande de dégradation. La protéine hybride synthétisée est donc stable. Ceci est confirmé par la résistance qu'elle montre aux conditions acides de purification sur colonne d'affinité.

La présence d'un pont disulfure entre les chaînes légères et lourdes hybrides, a été démontrée sur de l'hybride purifié. En effet, le dépôt de la protéine sur un gel SDS-PAGE non réducteur révèle la présence d'une bande protéique majoritaire de 97 kD, qui se dissocie complètement en conditions réductrices en deux bandes de 24 et 72 kD. L'ensemble de ces résultats, indique clairement que les bactéries transformées par le vecteur chargé (pLIP2/Fab), produisent dans leur périplasme un hybride composé d'une chaîne légère associée par un pont disulfure à la chaîne lourde PhoA.

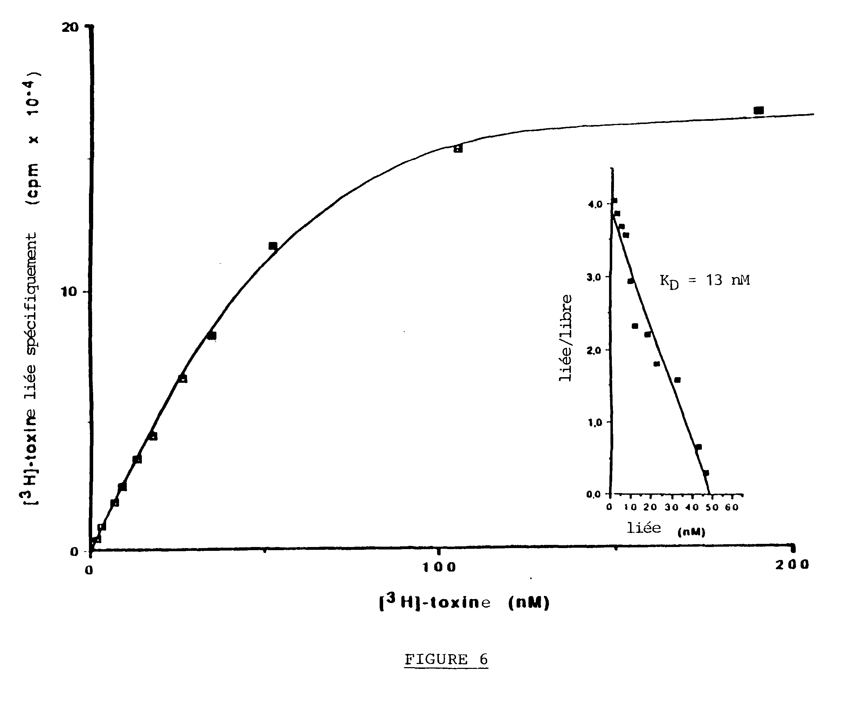

Les propriétés de liaison de l'hybride F(ab)₂-PhoA, ont été étudiées à partir de l'hybride purifié sur colonne d'affinité, (toxine alpha couplée à un gel de Sépharose® ). Des expériences radioimmunologiques ont été réalisées entre l'hybride purifié et des concentrations croissantes de toxine alpha tritiée (12 Ci/mmole) selon le protocole décrit précédemment [BOULAIN et al., Biochemistry 1982, 2910-2915]. La courbe de saturation obtenue (figure 6) indique que les hybrides F(ab)₂-PhoA lient spécifiquement et de manière saturable la toxine alpha tritiée. La représentation en Scatchard obtenue à partir de cette courbe (figure 6) donne une droite révélatrice d'une population homogène d'hybride. La constante de dissociation apparente déduite de la pente de la droite est de 13 nM, une valeur comparable aux 10 nM obtenus dans les mêmes conditions avec l'anticorps natif. En conclusion, la présence de PhoA à l'extrémité C-terminale du fragment F(ab)₂ ne perturbe ni l'association des deux chaînes de l'immunoglobuline, ni l'affinité du fragment d'anticorps pour sa cible.

### EXEMPLE 2 : Test immunoenzymatique mettant en oeuvre une protéine hybride conforme à l'invention.

Un test ELISA réalisé en utilisant des plaques sur lesquelles une toxine est adsorbée permet de mettre en évidence la fixation spécifique de la protéine hybride possédant l'activité enzymatique, sur la toxine.

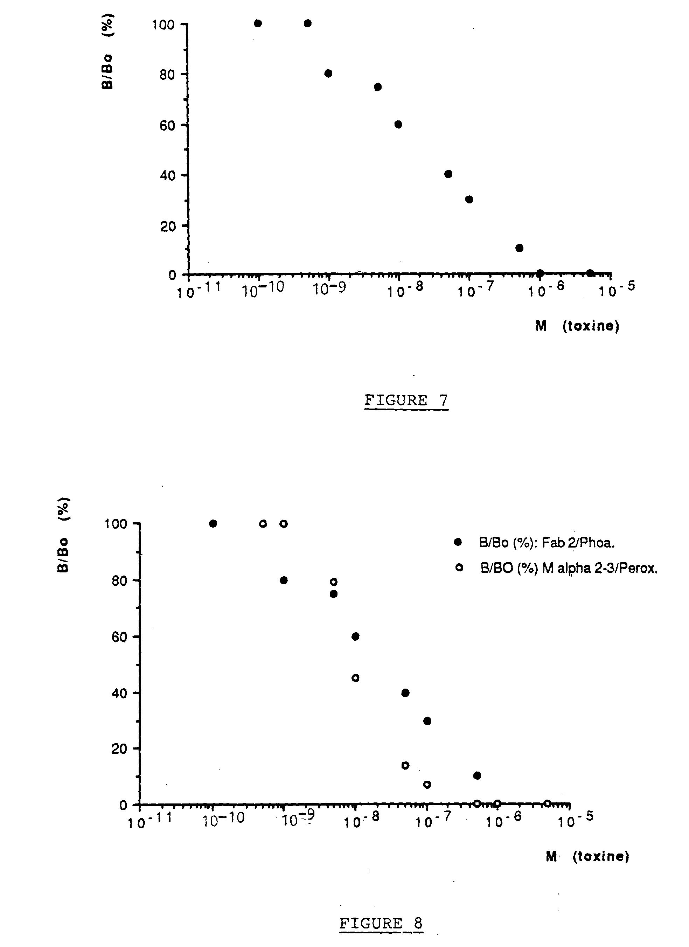

La figure 7 illustre la courbe d'inhibition de fixation de la protéine hybride conforme à l'invention F(ab)₂/PhoA, sur de la toxine α adsorbée (1 µg/ml) par des concentrations croissantes de toxine α libre. L'IC₅₀ déduite pour cette protéine hybride est de 2.10⁻⁸ M.

La figure 8 illustre une comparaison des courbes d'inhibition de fixation de ladite protéine hybride sur la toxine α adsorbée, par des concentrations croissantes de toxine libre, en utilisant soit la protéine hybride F(ab)₂/PhoA, soit un réactif Mα2-3/peroxydase, obtenu par couplage chimique classique. L'IC₅₀ obtenue pour ces deux réactifs est respectivement de 2.10⁻⁸ M et de 0,9.10⁻⁸ M ; ceci montre qu'une protéine hybride conforme à l'invention, présente en tant que réactif de diagnostic une sensibilité du même type que celle d'un réactif chimique, tout en présentant des avantages propres et notamment l'extrême simplicité de sa production, l'absence d'étapes longues et difficiles de purification, sa stabilité au cours de la production par la bactérie et également durant le test enzymatique, puisque l'activité enzymatique persiste pendant plus de 24 heures en présence de substrat.

### EXEMPLE 3 : Production d'un anticorps colorimétrique bivalent.

### 1) Caractéristiques et construction du gène hybride :

Un autre anticorps colorimétrique a été construit, entre la PhoA et un anticorps monoclonal murin (Mab17F12) de type IgG1/kappa, exclusivement spécifique des fragments Fab des immunoglobulines humaines de classe IgG.

Par rapport à l'exemple 1, cette seconde construction diffère et se caractérise par :
i) la sous-classe de la chaîne lourde de l'anticorps,
ii) la spécificité de l'anticorps qui présente un intérêt évident dans le domaine du diagnostic sérologique puisqu'il permet de détecter la présence d'anticorps humains de type IgG.

D'un point de vue pratique, le protocole suivi pour la construction de cet hybride, est identique à celui décrit dans l'exemple 1. Seules les amorces utilisées lors des expériences de PCR sont différentes et adaptées à la séquence de ce nouvel anticorps. Le plasmide chargé est appelé F(ab)₂/17F12/PhoA.

### 2) Caractérisation et propriétés de la protéine recombinante :

Dans les conditions appropriées, les bactéries transformées avec, soit le plasmide contenant le gène de la chaîne lourde hybride seul, soit le plasmide contenant le gène de la chaîne lourde hybride associé au gène de la chaîne légère, présentent une activité phosphatase et hydrolysent un substrat chromogène (X-P) sur boîte de Pétri. Des résultats identiques sont obtenus en solution avec le substrat pNPP.

La présence simultanée des deux chaînes de l'anticorps hybride (V_{H}-C_{H}1-PhoA et V_{L}-C_{L}), dans le périplasme des bactéries transformées par le vecteur chargé, est révélée par des expériences de marquage et d'immunoprécipitation similaires à celles décrites dans l'exemple 1.

Le profil protéique obtenu sur gel est rigoureusement identique à celui représenté dans la figure 5 de l'exemple 1.

L'ensemble de ces résultats démontre clairement que le système de production d'anticorps colorimétriques conforme à l'invention, est applicable à des immunoglobulines de sous-classes différentes.

### EXEMPLE 4 : Tests immunoenzymatiques de type ELISA, mettant en oeuvre la protéine hybride selon l'exemple 3.

La spécificité de l'hybride F(ab)₂/17F12/PhoA a été étudiée par test ELISA, en utilisant des plaques de microtitration sur lesquelles différentes classes et sous-classes d'immunoglobulines humaines et animales ont été adsorbées. Les sous-classes IgG1, 2, 3 ou 4 (associées à une chaîne légère lambda ou kappa), des IgM humaines ainsi que des IgG de rat, de chèvre et de lapin ont été testées. Le Tableau I ci-après compare les propriétés de l'anticorps monoclonal naturel à celles de l'hybride recombinant.

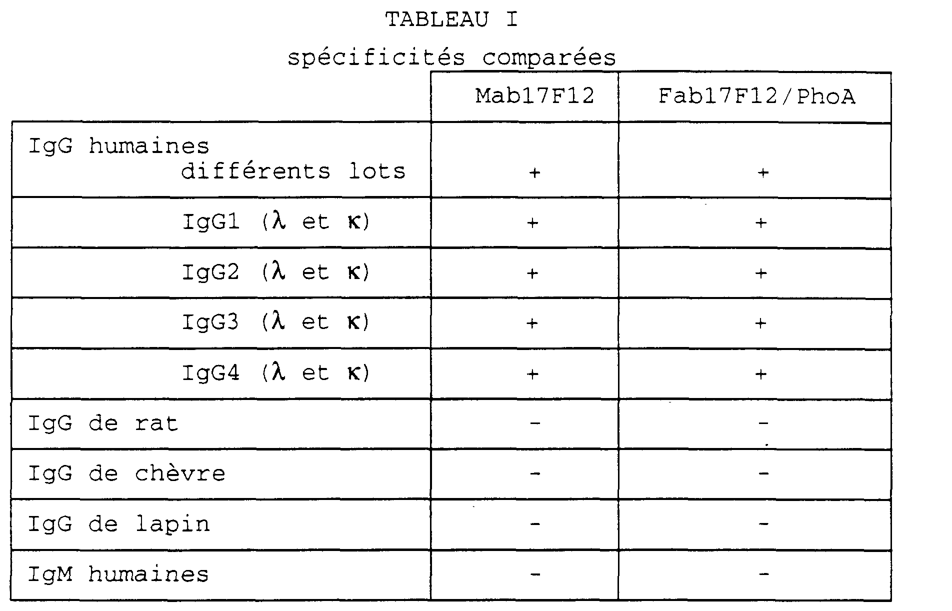

Ces résultats montrent la stricte conservation de la spécificité antigénique de l'anticorps exprimé chez la bactérie sous la forme d'un anticorps colorimétrique.

Un tel réactif présente donc un intérêt particulier en matière de diagnostic, puisqu'il est spécifique des immunoglobulines humaines de classe G.

Ce réactif est particulièrement intéressant dans les tests sérologiques basés sur la capture d'anticorps.

Le principe général de ces tests est le suivant : des anticorps sont spécifiquement piégés sur une phase solide portant un antigène. L'anticorps colorimétrique conforme à l'invention révèle ensuite la présence des anticorps capturés.

Cet exemple montre donc que l'invention permet le passage d'une sous-classe à l'autre d'anticorps ; en effet, un tel passage n'a pas révélé de problème particulier d'expression ni de stabilité de la molécule hybride.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

1. Protéine hybride du type comprenant un fragment d'immunoglobuline fusionné à une autre protéine P, caractérisée en ce qu'elle est constituée par une séquence hybride complexe comprenant :
. **une unité de base U constituée par :**
- deux chaînes peptidiques différentes SU₁ et SU₂, SU₁ comprenant les régions variables et constantes d'une chaîne légère d'une immunoglobuline et SU₂ comprenant au moins les régions variables et le domaine C_{H}1 d'une chaîne lourde d'une immunoglobuline, lesquelles chaînes peptidiques SU₁ et SU₂ sont reliées entre elles par au moins un pont disulfure, lesdites chaînes peptidiques SU₁ et SU₂ comprenant en amont de leur extrémité N-terminale, le fragment d'une protéine P₁ correspondant au signal d'exportation ou de sécrétion de ladite protéine P₁, exportée ou sécrétée par une cellule hôte unicellulaire choisie dans le groupe constitué par les souches bactériennes ou fungiques (*E. coli, Bacillus, Streptomyces*), les levures, les cellules de mammifère, les cellules végétales, ou les systèmes d'expression mixtes phage/cellule hôte comme les systèmes utilisant le baculovirus, et
- au moins un fragment fonctionnel d'une des deux sous-unités d'une protéine P dimérique comprenant le domaine nécessaire à la dimérisation de la protéine P, fusionnée par son extrémité N-terminale à l'une des chaînes peptidiques SU₁ ou SU₂,
. **laquelle unité de base U est associée à une autre unité de base U'**, identique ou différente de l'unité U, par l'intermédiaire des sous-unités de la protéine P ou d'un fragment de celles-ci.

2. Protéine hybride selon la revendication 1, caractérisée en ce que la protéine P₁ est différente de la protéine P.

3. Protéine hybride selon la revendication 1, caractérisée en ce que la protéine P1 est identique à la protéine P.

4. Protéine hybride selon l'une quelconque des revendications 1 à 3, caractérisée en ce que la protéine P dimérique est choisie dans le groupe qui comprend les protéines homodimériques formées de 2 sous-unités identiques et les protéines hétérodimériques formées de 2 sous-unités différentes.

5. Protéine hybride selon l'une quelconque des revendications 1 à 4, caractérisée en ce que ladite protéine hybride complexe comprend deux unités de base U et U' identiques ou différentes, constituées chacune :
- par une chaîne peptidique SU₁ comprenant les régions variables et constantes d'une chaîne légère d'une immunoglobuline appropriée (V_{L} + C_{L}), par une chaîne peptidique SU₂ comprenant les régions variables et le domaine C_{H}1 d'une chaîne lourde de la même immunoglobuline (VH + C_{H}1), lesquelles chaînes SU₁ et SU₂ sont reliées entre elles par au moins un pont disulfure,
- et par un fragment fonctionnel d'une phosphatase alcaline, comprenant le domaine nécessaire à la dimérisation de la protéine P, exportée ou excrétée par une cellule hôte appropriée, fusionnée par son extrémité, N-terminale à l'une des chaînes SU₁ ou SU₂, lesquelles unités de base U et U' sont réunies par l'assemblage des deux sous-unités de ladite phosphatase alcaline dimérique.

6. Unité de transcription T1 d'une protéine hybride complexe selon l'une quelconque des revendications 1 à 5, caractérisée en ce qu'elle comprend au moins successivement :
. un promoteur approprié,
. un premier site de fixation aux ribosomes (séquence de Shine-Dalgarno),
. une première séquence A comprenant une séquence signal d'une protéine P₁ exportée ou excrétée par une cellule hôte et susceptible de permettre l'exportation des immunoglobulines associées,
. une séquence C d'acide nucléique constitué par une séquence choisie dans le groupe constitué soit par une séquence codant au moins pour la région variable et le domaine C_{H}1 de la chaîne lourde d'une immunoglobuline, soit par une séquence codant pour la chaîne légère d'une immunoglobuline,
. une séquence D codant au moins pour un fragment fonctionnel comprenant le domaine nécessaire à la dimérisation de la protéine P, d'une sous-unité de la séquence mature d'une protéine P dimérique,
. au moins un codon stop,
. un deuxième site de fixation aux ribosomes (séquence de Shine-Dalgarno),
. une deuxième séquence A telle que définie ci-dessus,
. une séquence E d'acide nucléique codant pour la séquence de la chaîne non codée par la séquence C ci-dessus,
. un ou plusieurs codon stop,
. et un terminateur de transcription.

7. Unité de transcription selon la revendication 6, caractérisée en ce qu'elle comprend en outre, entre la première séquence A et la séquence C et entre la deuxième séquence A et la séquence E ci-dessus, une séquence B d'acide nucléique codant pour un fragment NH₂-terminal de la protéine P₁ mature.

8. Unité de transcription selon la revendication 7, caractérisée en ce que P₁ et P sont identiques.

9. Unité de transcription selon la revendication 8, caractérisée en ce que P est choisie dans le groupe qui comprend les protéines homodimériques formées de 2 sous-unités identiques et les protéines hétérodimériques formées de 2 sous-unités différentes.

10. Unité de transcription selon l'une quelconque des revendications 7 à 9, caractérisée en ce qu'elle est constituée par :
. un promoteur approprié,
. un premier site de fixation aux ribosomes (séquence de Shine-Dalgarno),
. une première séquence A comprenant une séquence signal du gène de structure d'une protéine homo- ou hétérodimérique exportée ou sécrétée par une cellule hôte,
. une séquence B comprenant une séquence d'acide nucléique codant pour un fragment NH₂-terminal de la protéine homo- ou hétérodimérique mature,
. une séquence C constituée par une séquence choisie dans le groupe constitué soit par une séquence d'acide nucléique codant pour la région variable et le domaine C_{H}1 de la chaîne lourde d'une immunoglobuline, soit par une séquence codant pour la chaîne légère d'une immunoglobuline,
. une séquence D constituée par un fragment codant pour au moins un fragment fonctionnel comprenant le domaine nécessaire à la dimérisation de la protéine P, de la séquence mature de ladite protéine P homo- ou hétérodimérique,
. au moins un codon stop,
. un deuxième site de fixation aux ribosomes,
. une deuxième séquence A telle que définie ci-dessus,
. une séquence B comprenant une séquence d'acide nucléique codant pour un fragment NH₂-terminal de la protéine homo- ou hétérodimérique mature,
. une séquence E constituée par une séquence d'acide nucléique codant pour la chaîne de ladite immunoglobuline non codée par la séquence C ci-dessus,
. au moins un codon stop,
. et un terminateur de transcription,
l'ensemble de ces fragments étant en phase de lecture et codant pour la protéine hybride complexe selon l'une quelconque des revendications 1 à 5, présentant à la fois les propriétés de l'enzyme et certaines propriétés des fragments Fab des immunoglobulines.

11. Unité de transcription selon la revendication 10, caractérisée en ce que ladite protéine homo- ou hétérodimérique est une phosphatase alcaline.

12. Unité de transcription selon la revendication 11, caractérisée en ce qu'elle est constituée par :
. un promoteur approprié,
. un premier site de fixation aux ribosomes (séquence de Shine-Dalgarno),
. une première séquence A comprenant la séquence signal du gène de structure de la phosphatase alcaline,
. un fragment B codant pour les 6 aminoacides N-terminaux de la phosphatase alcaline mature,
. une séquence C constituée par une séquence d'acide nucléique choisie dans le groupe constitué soit par une séquence codant pour la région variable et le domaine C_{H}1 de la chaîne lourde d'une immunoglobuline, soit par une séquence codant pour la chaîne légère d'une immunoglobuline,
. une séquence D constituée par un fragment codant pour les 444 aminoacides restants C-terminaux de la séquence mature de ladite enzyme dimérique,
. au moins un codon stop,
. un deuxième site de fixation aux ribosomes,
. une deuxième séquence A telle que définie ci-dessus,
. un fragment B codant pour les 6 aminoacides N-terminaux de la phosphatase alcaline mature,
. une séquence E d'acide nucléique telle que définie ci-dessus codant pour la chaîne non codée par la séquence C ci-dessus, de ladite immunoglobuline,
. au moins un codon stop,
. et un terminateur de transcription.

13. Unité de transcription selon la revendication 12, caractérisée en ce qu'elle comprend en outre, notamment pour permettre l'insertion des fragments codant pour les immunoglobulines et pour la mise en phase de lecture de l'ensemble de ces fragments, en amont et/ou en aval de la séquence C et en amont et/ou en aval de la séquence E, un fragment d'acide nucléique appropriée, laquelle unité de transcription, en phase de lecture, code pour une protéine hybride selon l'une quelconque des revendications 1 à 5.

14. Unité de transcription selon la revendication 13, caractérisée en ce qu'elle comprend une première séquence A signal du gène de structure de la phosphatase alcaline, une première séquence B codant pour les 6 aminoacides N-terminaux de la phosphatase alcaline, un site de restriction Bgl II, une séquence C, choisie dans le groupe constitué soit par une séquence codant pour la région variable et le domaine C_{H}1 d'une chaîne lourde d'une immunoglobuline, soit par la séquence codant pour la chaîne légère d'une immunoglobuline, un site de restriction Sal I, un fragment D codant pour les 444 aminoacides restants C-terminaux de la phosphatase alcaline, une deuxième séquence A, une deuxième séquence B, un site de restriction Sac I, une séquence E codant pour la séquence non codée par la séquence C, un site de restriction XbaI, au moins un codon stop et un terminateur de transcription.

15. Unité de transcription T2, caractérisée en ce qu'elle comprend :
. un promoteur approprié,
. un site de fixation aux ribosomes (séquence de Shine-Dalgarno),
. une première séquence A signal d'un gène de structure d'une protéine P₁, une séquence D apte à coder pour un fragment fonctionnel de la séquence mature d'une protéine P dimérique et un ou plusieurs sites de restriction uniques, situés à la jonction entre la première séquence A et la séquence D et apte à recevoir une séquence C choisie dans le groupe constitué, soit par une séquence codant pour la région variable et le domaine C_{H}1 d'une chaîne lourde d'une immunoglobuline, soit par la séquence codant pour la chaîne légère d'une immunoglobuline,
. au moins un codon stop,
. un site de fixation aux ribosomes (séquence de Shine-Dalgarno),
. une deuxième séquence A telle que d,finie ci-dessus et un ou plusieurs sites de restriction uniques, situés en aval de ladite séquence A et aptes à recevoir une séquence E codant pour la séquence non codée par la séquence C,
. au moins un codon stop,
. et un terminateur de transcription.

16. Unité de transcription T2 selon la revendication 15, caractérisée en ce qu'elle comprend :
. un promoteur approprié,
. un site de fixation aux ribosomes (séquence de Shine-Dalgarno),
. une première séquence A signal d'un gène de structure d'une protéine P₁,
. une première séquence B codant pour un fragment NH₂-terminal d'une protéine P₁ mature, une séquence D apte à coder pour une protéine P mature ou un fragment de celle-ci et un ou plusieurs sites de restriction uniques, situés à la jonction entre le fragment NH₂-terminal de séquence codant pour la protéine P₁ et la séquence codant pour la protéine P et aptes à recevoir une séquence C telle que définie ci-dessus,
. au moins un codon stop,
. un site de fixation aux ribosomes (séquence de Shine-Dalgarno),
. une deuxième séquence A telle que définie ci-dessus,
. une deuxième séquence B telle que définie ci-dessus et un ou plusieurs sites de restriction uniques, situés en aval de ladite séquence A et aptes à recevoir une séquence E telle que définie ci-dessus,
. au moins un codon stop,
. et un terminateur de transcription.

17. Unité de transcription selon la revendication 16, caractérisée en ce que P₁ et P sont identiques.

18. Unité de transcription selon l'une quelconque des revendications 6 à 16, caractérisée en ce que le promoteur est choisi dans le groupe qui comprend le promoteur du gène phoA et tout autre promoteur.

19. Vecteurs d'expression et/ou de clonage d'une protéine hybride complexe selon l'une quelconque des revendications 1 à 5, caractérisés en ce que chaque vecteur inclut une unité de transcription T1 selon l'une quelconque des revendications 6 à 14 ou la revendication 18, insérée dans une structure génétique appropriée, notamment choisie dans le groupe qui comprend des plasmides, des phages, des cosmides ou des chromosomes appropriés.

20. Vecteur selon la revendication 19, caractérisé en ce que la structure génétique est un plasmide, et l'unité de transcription T1 comprend un gène ou un fragment de gène codant pour une protéine P₁, identique à la protéine P, laquelle protéine est une enzyme dimérique.

21. Vecteurs potentiels d'expression et/ou de clonage d'une protéine hybride complexe selon l'une quelconque des revendications 1 à 5, caractérisés en ce que chaque vecteur inclut une unité de transcription T2 selon l'une quelconque des revendications 15 à 18, insérée dans une structure génétique appropriée, notamment choisie dans le groupe qui comprend des plasmides, des phages, des cosmides ou des chromosomes appropriés.

22. Microorganisme obtenu par transformation génétique, caractérisé en ce qu'il est obtenu par modification appropriée d'une souche d'*E. coli* par un vecteur selon l'une quelconque des revendications 19 à 21 et notamment par transformation.

23. Microorganisme selon la revendication 22, caractérisé en ce qu'il est avantageusement une souche CC118 d'*E. coli* transformée par un vecteur non chargé selon la revendication 21.

24. Microorganisme selon la revendication 22 ou la revendication 23, caractérisé en ce qu'il a été déposé en date du 31 janvier 1992, sous le numéro I-1168 auprès de la Collection Nationale des Cultures de Microorganismes tenue par l'Institut Pasteur.

25. Réactif de diagnostic, caractérisé en ce qu'il est constitué par une protéine hybride complexe selon l'une quelconque des revendications 1 à 5.

26. Procédé de dosage immunoenzymatique de protéines, caractérisé en ce qu'il consiste :
- à mettre en contact un échantillon biologique convenable avec un réactif de diagnostic selon la revendication 25 et
- à détecter ledit réactif de diagnostic sous forme de complexe et/ou sous forme libre par une réaction colorimétrique appropriée.

27. Trousse prête à l'emploi pour la mise en oeuvre du dosage immunoenzymatique selon la revendication 25, caractérisée ne ce qu'elle comprend outre les quantités appropriées de tampons et réactifs utiles pour la mise en oeuvre du dosage, des quantités appropriées du réactif selon la revendication 25.

28. Agent à visée thérapeutique, caractérisé en ce qu'il est constitué par une protéine hybride complexe selon l'une quelconque des revendications 1 à 5.

29. Agent thérapeutique selon la revendication 28, caractérisé en ce que la protéine P est une substance cytotoxique, et les fragments d'immunoglobuline associés dans les unités de base sont d'origine humaine et présentent une spécificité vis-à-vis d'une cellule cible, préalablement sélectionnée.

30. Procédé de production d'anticorps bivalents et bispécifiques, caractérisé en ce que :
- on produit, dans un vecteur selon l'une quelconque des revendications 19 et 20, deux unités de transcription T1 dont les parties codant pour les domaines d'immunoglobulines sont différentes, lesquelles unités sont sous le contrôle du même promoteur et du même terminateur de transcription, puis, si nécessaire,
- on sélectionne les hybrides produits pour lesquels l'unité de base U est différente de l'unité de base U', notamment par chromatographie d'affinité.

## Patentansprüche

1. Hybridprotein des Typs, der ein Immunoglobulin-Fragment an ein anderes Protein P gebunden umfaßt,
dadurch **gekennzeichnet**, daß
es durch eine komplexe Hybridsequenz gebildet wird, die umfaßt:
eine Basiseinheit U, bestehend aus:
- zwei unterschiedlichen Peptidketten SU₁ und SU₂, wobei SU₁ die variablen und konstanten Regionen einer leichten Kette eines Immunoglobulins umfaßt, und SU₂ mindestens die variablen Regionen und die Domäne C_{H}1 einer schweren Kette eines Immunoglobulins umfaßt; die Peptidketten SU₁ und SU₂ durch mindestens eine Dislufid-Brücke miteinander verbunden sind; die Peptidketten SU₁ und SU₂ stromaufwärts ihres N-terminalen Endes das Fragment eines Proteins P₁ enthalten, das einem Ausscheidungs- oder Sekretionssignal des Proteins P₁ entspricht, das durch eine einzellige Wirtszelle, ausgewählt aus der Gruppe bestehend aus Bakterien- und Pilzstämmen (E. coli, Bacillus, Streptomyces), Hefen, Säugetierzellen, Pflanzenzellen, oder durch die gemischten Expressionssysteme Phage/Wirtszelle wie die Systeme, die das Baculovirus verwenden, ausgeschieden oder sekretiert wird, und
- mindestens einem funktionellen Fragment einer der zwei Untereinheiten eines dimeren Proteins P, das die zur Dimerisierung des Proteins P notwendige Domäne umfaßt und das durch sein N-terminales Ende an eine der Peptidkette SU₁ oder SU₂ gebunden ist;
wobei die Basiseinheit U mit Hilfe von Untereinheiten des Proteins P oder eines Fragments dieser mit einer anderen Basiseinheit U', die mit der Einheit U identisch oder von dieser verschieden ist, verbunden ist.

2. Hybridprotein nach Anspruch 1,
dadurch **gekennzeichnet**, daß
das Protein P₁ vom Protein P verschieden ist.

3. Hybridprotein nach Anspruch 1,
dadurch **gekennzeichnet**, daß
das Protein P₁ mit Protein P identisch ist.

4. Hybridprotein nach einem der Ansprüche 1 bis 3,
dadurch **gekennzeichne**t, daß
das dimere Protein P aus der Gruppe, die die homodimeren Proteine, gebildet aus zwei identischen Untereinheiten, und die heterodimeren Proteine, gebildet aus zwei unterschiedlichen Einheiten, umfaßt, ausgewählt ist.

5. Hybridprotein nach einem der Ansprüche 1 bis 4,
dadurch **gekennzeichnet**, daß
das komplexe Hybridprotein zwei Basiseinheiten U und U', die identisch oder unterschiedlich sind, umfaßt, jede bestehend aus
- einer Peptidkette SU₁, die die variablen und konstanten Regionen einer leichten Kette eines geeigneten Immunoglobulins (V_{L} + C_{L}) umfaßt, einer Peptidkette SU₂, die die variablen Regionen und die Domäne C_{H}1 einer schweren Kette desselben Immunoglobulins (VH + C_{H}1) umfaßt, wobei die Ketten SU₁ und SU₂ mindestens durch eine Disulfidbrücke miteinander verbunden sind,
- und einem funktionellen Fragment einer alkalischen Phosphatase, das die Domäne umfaßt, die für die Dimerisierung des Proteins P notwendig ist, das durch eine geeignete Wirtszelle ausgeschieden oder sekretiert wird, und mit seinem N-terminalen Ende an eine der Ketten SU₁ oder SU₂ gebunden ist, wobei die Basiseinheiten U und U' durch Zusammenfügen der zwei Untereinheiten der dimeren alkalischen Phosphatase verbunden werden.

6. Transkriptionseinheit T1 eines komplexen Hybridproteins nach einem der Ansprüche 1 bis 5,
dadurch **gekennzeichnet**, daß
sie nacheinander mindestens umfaßt:
- einen geeigneten Promotor,
- eine erste Bindungsstelle für Ribosomen (Shine-Dalgarno-Sequenz),
- eine erste Sequenz A, die eine Signalsequenz eines Proteins P₁ umfaßt, das von einer Wirtszelle ausgeschieden oder sekretiert wird, und geeignet ist, eine Ausscheidung der assoziierten Immunoglobuline zuzulassen,
- eine Nucleinsäuresequenz C, bestehend aus einer Sequenz, die aus der Gruppe bestehend aus einer Sequenz, die mindestens für die variable Region und die Domäne C_{H}1 der schweren Kette eines Immunoglobulins codiert, und einer Sequenz, die für die leichte Kette eines Immunoglobulins codiert, ausgewählt ist;
- eine Sequenz D, die mindestens für ein funktionelles Fragment mit einer Untereinheit der reifen Sequenz eines dimeren Proteins P codiert, wobei das Fragment die Domäne umfaßt, die für die Dimerisierung des Proteins P, notwendig ist;
- mindestens ein Stopp-Codon;
- eine zweite Bindungsstelle für Ribosomen (Shine-Dalgarno-Sequenz);
- eine zweite Sequenz A, wie die oben definierte;
- eine Nucleinsäuresequenz E, die für die Sequenz der Kette codiert, die durch die obige Sequenz C nicht codiert wird;
- ein oder mehrere Stopp-Codons und
- einen Transkriptionsterminator.

7. Transkriptionseinheit nach Anspruch 6,
dadurch **gekennzeichnet**, daß
sie außerdem zwischen der ersten Sequenz A und der Sequenz C und zwischen der zweiten Sequenz A und der Sequenz E eine Nucleinsäuresequenz B umfaßt, die für ein NH₂-terminales Fragment des reifen Proteins P₁ codiert.

8. Transkriptionseinheit nach Anspruch 7,
dadurch **gekennzeichnet**, daß
P₁ und P identisch sind.

9. Transkriptionseinheit nach Anspruch 8,
dadurch **gekennzeichnet**, daß
P aus der Gruppe, die die homodimeren Proteine, gebildet aus zwei identischen Untereinheiten, und die heterodimeren Proteine, gebildet aus zwei unterschiedlichen Untereinheiten, umfaßt, ausgewählt ist.

10. Transkriptionseinheit nach einem der Ansprüche 7 bis 9,
dadurch **gekennzeichnet**, daß
sie umfaßt:
- einen geeigneten Promotor;
- eine erste Bindungsstelle für Ribosomen (Shine-Dalgarno-Sequenz);
- eine erste Sequenz A, die eine Signalsequenz des Strukturgens eines homodimeren oder heterodimeren Proteins, das durch eine Wirtszelle ausgeschieden oder sekretiert wird, umfaßt;
- eine Sequenz B, die eine Nucleinsäuresequenz umfaßt, die für ein NH₂-terminales Fragment des reifen homo- oder heterodimeren Proteins codiert;
- eine Sequenz, bestehend aus einer Sequenz, die aus der Gruppe bestehend aus einer Nucleinsäuresequenz, die für die variable Region und die Domäne C_{H}1 der schweren Kette eines Immunoglobulins codiert, und einer Sequenz, die für die leichte Kette eines Immunoglobulins codiert, ausgewählt ist;
- eine Sequenz D, bestehend aus einem Fragment, das für mindestens ein funktionelles Fragment der reifen Sequenz des homo- oder heterodimeren Proteins P codiert, wobei das Fragment die Domäne umfaßt, die zur Dimerisierung des Proteins P notwendig ist;
- mindestens ein Stopp-Codon;
- eine zweite Bindungsstelle für Ribosomen;
- eine zweite Sequenz A wie die oben definierte;
- eine Sequenz B, die eine Nucleinsäuresequenz umfaßt, die für ein NH₂-terminales Fragment des reifen homo- oder heterodimern Proteins codiert;
- eine Sequenz E, bestehend aus einer Nucleinsäuresequenz, die für die Kette des Immunoglobulins codiert, die nicht durch die obige Sequenz C codiert wird;
- mindestens ein Stopp-Codon; und
- einen Transkriptionsterminator;
wobei die Gesamtheit dieser Fragmente in der Lesephase ist und für das komplexe Hybridprotein nach einem der Ansprüche 1 bis 5 codiert, die Eigenschaften des Enzyms und bestimmte Eigenschaften der Fragmente Fab der Immunoglobuline auf einmal aufweist.

11. Transkriptionseinheit nach Anspruch 10,
dadurch **gekennzeichnet**, daß
das homodimere oder heterodimere Protein eine alkalische Phosphatase ist.

12. Transkriptionseinheit nach Anspruch 11,
dadurch **gekennzeichnet**, daß
sie umfaßt:
- einen geeigneten Promotor;
- eine erste Bindungsstelle für Ribosomen (Shine-Dalgarno-Sequenz);
- eine erste Sequenz A, die die Signalsequenz des Strukturgens der alkalischen Phosphatase umfaßt;
- ein Fragment B, das für die 6 N-terminalen Aminosäuren der reifen alkalischen Phosphatase codiert;
- eine Sequenz C, bestehend aus einer Nucleinsäuresequenz, die aus der Gruppe bestehend aus einer Sequenz, die für die variable Region und die Domäne C_{H}1 der schweren Kette eine Immunoglobulins codiert, und einer Sequenz, die für die leichte Kette eines Immunoglobulins codiert, ausgewählt ist;
- eine Sequenz D, bestehend aus einem Fragment, das für die 444 C-terminalen Aminosäure-Reste der reifen Substanz des dimeren Enzyms codiert;
- mindestens ein Stopp-Codon;
- eine zweite Bindungsstelle für Ribosomen;
- eine zweite Sequenz A wie die oben definierte;
- ein Fragment B, das für die 6 N-terminalen Aminosäuren der reifen alkalischen Phosphatase codiert;
- eine Nucleinsäuresequenz E wie die oben definierte, die für die Kette des Immunoglobulins codiert, die nicht durch die obige Sequenz C codiert wird;
- mindestens eine Stopp-Codon; und
- einen Transkriptionsterminator.

13. Transkriptionseinheit nach Anspruch 12,
dadurch **gekennzeichnet,** daß
sie außerdem - insbesondere zur Ermöglichung der Insertion der Fragmente, die für die Immunoglobuline codieren, und zur Durchführung des Lesens der Gesamtheit dieser Fragmente - stromaufwärts und/oder stromabwärts der Sequenz C und stromaufwärts und/oder stromabwärts der Sequenz E, ein geeignetes Nucleinsäurefragment umfaßt, wobei die Transkriptionseinheit im Lesezustand für ein Hybridprotein nach einem der Ansprüche 1 bis 5 codiert.

14. Transkriptionseinheit nach Anspruch 13,
dadurch **gekennzeichnet**, daß
sie eine erste Signalsequenz A des Strukturgens der alkalischen Phosphatase, eine erste Sequenz B, die für die 6 N-terminalen Aminosäuren der alkalischen Phosphatase codiert, eine Restriktionsstelle Bgl II, eine Sequenz C, ausgewählt aus der Gruppe bestehend aus einer Sequenz, die für die variable Region und die Domäne C_{H}1 einer schweren Kette eines Immunoglobulins codiert, und der Sequenz, die für die leichte Kette eines Immunoglobulins codiert, eine Restriktionsstelle Sal I, ein Fragment D, das sie für die 444 C-terminalen Aminosäuren der alkalischen Phosphatase codiert, eine zweite Sequenz A, eine zweite Sequenz B, eine Restriktionsstelle Sac I, eine Sequenz E, die für die Sequenz codiert, die nicht durch die Sequenz C codiert wird, eine Restriktionsstelle XbaI, mindestens ein Stopp-Codon und einen Transkriptionsterminator umfaßt.

15. Transkriptionseinheit T2,
dadurch **gekennzeichnet**, daß sie umfaßt:
- einen geeigneten Promotor;
- eine Bindungsstelle für Ribosomen (Shine-Dalgarno-Sequenz);
- eine erste Signalsequenz A eines Strukturgens eines Proteins P₁, eine Sequenz D, die fähig ist, für ein funktionelles Fragment der reifen Sequenz eines dimeren Proteins P zu codieren, und eine oder mehrere einzelne Restriktionsstellen, die an der Verbindungsstelle zwischen der ersten Sequenz A und der Sequenz D gelegen sind und eine Sequenz C aufnehmen können, die aus der Gruppe bestehend aus einer Sequenz, die für die variable Region und die Domäne C_{H}1 einer schweren Kette eines Immunoglobulins codiert, und einer Sequenz, die für die leichte Kette eine Immunoglobulins codiert, ausgewählt ist;
- mindestens ein Stopp-Codon;
- eine Bindungsstelle für Ribosomen (Shine-Dalgarno-Sequenz);
- eine zweite Sequenz A wie oben definiert und eine oder mehrere einzelne Restriktionsstellen, die stromabwärts der Sequenz A gelegen sind und eine Sequenz E aufnehmen können, die für die Sequenz codiert, die nicht durch die Sequenz C codiert sind;
- mindestens ein Stopp-Codon;
- und einen Transkriptionsterminator.

16. Transkriptionseinheit T2 nach Anspruch 15,
dadurch **gekennzeichnet,** daß sie umfaßt:
- einen geeigneten Promotor;
- eine Bindungsstelle für Ribosomen (Shine-Dalgarno-Sequenz);
- eine erste Signalsequenz A eines Strukturgens eines Proteins P₁;
- eine erste Sequenz B, die für ein NH₂-terminales Fragment eines reifen Proteins P₁ codiert, eine Sequenz D, die fähig ist, für ein reifes Protein P oder ein Fragment desselben zu codieren, und eine oder mehrere einzelne Restriktionsstellen, die an der Verbindungsstelle zwischen dem NH₂-terminalen Fragment der Sequenz, die für das Protein P₁ codiert, und der Sequenz, die für das Protein P codiert, gelegen sind und eine Sequenz C, wie sie oben definiert ist, aufnehmen können;
- mindestens ein Stopp-Codon;
- eine Bindungsstelle für Ribosomen (Shine-Dalgarno-Sequenz);
- eine zweite Sequenz A, wie sie oben definiert ist;
- eine zweite Sequenz B, wie sie oben definiert ist, und eine oder mehrere einzelne Restriktionsstellen, die stromabwärts der Sequenz A gelegen sind und eine Sequenz E, wie sie oben definiert ist, aufnehmen können;
- mindestens ein Stopp-Codon; und
- einen Transkirptionsterminator.

17. Transkriptionseinheit nach Anspruch 16,
dadurch **gekennzeichnet,** daß
P₁ und P identisch sind.

18. Transkriptionseinheit nach einem der Ansprüche 6 bis 16,
dadurch **gekennzeichnet**, daß
der Promotor aus der Gruppe, die den Promotor des Gens phoA und jeden anderen Promotor umfaßt, ausgewählt ist.

19. Vektoren zur Expression und/oder Klonierung eines komplexen Hybridproteins nach einem der Ansprüche 1 bis 5,
dadurch **gekennzeichnet**, daß
jeder Vektor eine Transkriptionseinheit T1 nach einem der Ansprüche 6 bis 14 oder nach Anspruch 18 in eine geeignete genetische Struktur, die insbesondere aus der Gruppe bestehend aus geeigneten Plasmiden, Phagen, Cosmiden und Chromosomen ausgewählt ist, insertiert enthält.

20. Vektor nach Anspruch 19,
dadurch **gekennzeichnet**, daß
die genetische Struktur ein Plasmid ist und die Transkriptionseinheit T1 ein Gen oder ein Fragment des Gens, das für ein Protein P₁, identisch mit Protein P, codiert, umfaßt, wobei das Protein ein dimeres Enzym ist.

21. Vektoren, die zur Expression und/oder Klonierung eines komplexen Hybridproteins nach einem der Ansprüche 1 bis 5 fähig sind,
dadurch **gekennzeichnet**, daß
jeder Vektor eine Transkriptionseinheit T2 nach einem der Ansprüche 15 bis 18 in eine geeignete genetische Struktur, die insbesondere aus der Gruppe bestehend aus geeigneten Plasmiden, Phagen, Cosmiden und Chromosomen ausgewählt ist, insertiert enthält.

22. Mikroorganismus, der durch genetische Transformation erhalten wird,
dadurch **gekennzeichnet**, daß
er durch geeignete Modifikation eines Stamms von E. coli durch einen Vektor nach einem der Ansprüche 19 bis 21 und insbesondere durch Transformation erhalten wird.

23. Mikroorganismus nach Anspruch 22,
dadurch **gekennzeichnet**, daß
er vorteilhafterweise Stamm CC118 von E. coli, der durch einen nicht-beladenen Vektor nach Anspruch 21 transformiert ist, ist.

24. Mikroorganismus nach Anspruch 22 oder Anspruch 23,
dadurch **gekennzeichnet**, daß
er am 31. Januar 1992 unter der Nr. I-1168 bei der Collection Nationale des Culture de Microorganismes, geführt vom l'Institut Pasteur, hinterlegt wurde.

25. Diagnosereagens,
dadurch **gekennzeichnet**, daß
es aus einem komplexen Hybridprotein nach einem der Ansprüche 1 bis 5 besteht.

26. Verfahren zur immunenzymatischen Dosierung von Proteinen,
dadurch **gekennzeichnet**, daß
es darin besteht, daß
- eine zweckdienliche biologische Probe mit einem Diagnosereagens nach Anspruch 25 in Kontakt gebracht wird und
- das Diagnosereagens in Form eines Komplexes und/oder in freier Form durch geeignete kolorimetrische Reaktion nachgewiesen wird.

27. Kasten, gebrauchsfertig zur Verwendung bei der immunenzymatischen Dosierung gemäß Anspruch 25,
dadurch **gekennzeichnet**, daß
er außer den geeigneten Mengen an Puffern und Reagenzien, die zur Durchführung der Dosierung nützlich sind, geeignete Mengen des Reagenzes nach Anspruch 25 enthält.

28. Therapeutisches Mittel,
dadurch **gekennzeichnet,** daß
es aus einem komplexen Hybridprotein nach einem der Ansprüche 1 bis 5 besteht.

29. Therapeutisches Mittel nach Anspruch 28,
dadurch **gekennzeichnet**, daß
das Protein P eine cytotoxische Substanz ist und die Immunoglobulin-Fragmente, die in den Basiseinheiten gebunden sind, menschlichen Ursprungs sind und gegenüber einer Zielzelle, die vorher ausgewählt wurde, eine besondere Eigenschaft aufweisen.

30. Verfahren zur Herstellung von bivalenten und bispezifischen Antikörpern,
dadurch **gekennzeichnet**, daß
- man in einem Vektor nach einem der Ansprüche 19 und 20 zwei Transkriptionseinheiten T1, deren Teile, die für die Domänen von Immunoglobulinen codieren, unterschiedlich sind produziert, wobei die Einheiten unter Steuerung desselben Promotors und desselben Transkriptionsterminators sind, und dann selektioniert man, wenn notwendig,
- die Hybridprodukte, für die die Basiseinheit U sich von der Basiseinheit U' unterscheidet, insbesondere durch Affinitätschromatographie

## Claims

1. Hybrid protein of the type comprising an immunoglobulin fragment attached to another protein P, characterized in that it is composed of a complex hybrid sequence comprising:
* **one base unit U composed of:**
- two different peptide chains SU₁ and SU₂, SU₁ comprising variable and constant regions of a light chain of an immunoglobulin and SU₂ comprising at least the variable regions and the C_{H}1 domain of a heavy chain of an immunoglobulin, these peptide chains SU₁ and SU₂ being linked together by at least one disulphide bridge, the said peptide chains SU₁ and SU₂ comprising, upstream of their N-terminal end, the fragment of a protein P₁ corresponding to the exportation or secretion signal of the said protein P₁, exported or secreted by a unicellular host cell chosen from among the group consisting of bacterial or fungal strains (*E. coli, Bacillus, Streptomyces),* yeasts, mammalian cells, plant cells or mixed expression phage/cell host systems such as the systems using baculovirus, and
- at least one functional fragment of one of the two sub-units of a dimeric protein P comprising the domain necessary for the dimerisation of the protein P, linked by its N-terminal end to one of the peptide chains SU₁ or SU₂,
• **the said base unit U is attached to another base unit U**', the same as or different to the unit U, through the intermediary of sub-units of the protein P or of a fragment of the latter.

2. Hybrid protein according to Claim 1, characterized in that the protein P₁ is different to protein P.

3. Hybrid protein according to Claim 1, characterized in that the protein P₁ is identical to protein P.

4. Hybrid protein according to any one of the Claims 1 to 3, characterized in that the dimeric protein P is chosen from among the group comprising the homodimeric proteins formed from 2 identical sub-units and the heterodimeric proteins formed from 2 dissimilar sub-units.

5. Hybrid protein according to any one of the Claims 1 to 4, characterized in that the said complex hybrid protein comprises two base units U and U', identical or different, each consisting:
- of a peptide chain SU₁ comprising the variable and constant regions of a light chain of an appropriate immunoglobulin (V_{L} + C_{L}) and of a peptide chain SU₂ comprising the variable regions and the C_{H}1 domain of a heavy chain of the same immunoglobulin (VH + C_{H}1), the said chains SU₁ and SU₂ being linked to one another by at least one disulphide bridge,
- and of a functional fragment of an alkaline phosphatase comprising the domain necessary for the dimerisation of the protein P, exported or excreted by an appropriate host cell, attached by its N-terminal end to one of the chains SU₁ or SU₂, these base units U et U' being joined together by the assembly of two sub-units of the said dimeric alkaline phosphatase.

6. Transcription unit T1 of a complex hybrid protein according to any one of the Claims 1 to 5, characterized in that it comprises at least, in succession:
- an appropriate promoter,
- a first ribosome binding site (Shine-Dalgarno sequence),
- a first sequence A comprising a signal sequence of a protein P₁ exported or excreted by a host cell and capable of allowing the export of the linked immunoglobulins,
- a nucleic acid sequence C consisting of a sequence chosen from the group consisting of either a sequence coding for at least the variable region and the C_{H}1 domain of the heavy chain of an immunoglobulin, or of a sequence coding for the light chain of an immunoglobulin,
- a sequence D coding for at least a functional fragment comprising the domain necessary for the dimerisation of the protein P, of one sub-unit of the mature sequence of a dimeric protein P,
- at least one stop codon,
- a second ribosome binding site (Shine-Dalgarno sequence),
- a second sequence A as defined above,
- a nucleic acid sequence E coding for the sequence of the chain not coded by the sequence C above,
- one or more stop codons,
- and a transcription terminator.

7. Transcription unit according to Claim 6, characterized in that it also comprises, between the first sequence A and the sequence C and between the second sequence A and the sequence E above, a nucleic acid sequence B coding for an NH₂-terminal fragment of the mature protein P₁.

8. Transcription unit according to Claim 7, characterized in that P₁ and P are identical.

9. Transcription unit according to Claim 8, characterized in that P is chosen from among the group comprising the homodimeric proteins formed from 2 identical sub-units and the heterodimeric proteins formed from 2 dissimilar sub-units.

10. Transcription unit according to any one of the Claims 7 to 9, characterized in that it is composed of:
- an appropriate promoter,
- a first ribosome binding site (Shine-Dalgamo sequence),
- a first sequence A comprising a signal sequence of the structure gene of a homo- or heterodimeric protein exported or secreted by a host cell,
- a sequence B comprising a nucleic acid sequence coding for an NH₂-terminal fragment of the mature homo- or heterodimeric protein,
- a sequence C consisting of a sequence chosen from among the group composed of either a nucleic acid sequence coding for the variable region and the C_{H}1 domain of the heavy chain of an immunoglobulin, or of a sequence coding for the light chain of an immunoglobulin,
- a sequence D consisting of a fragment coding for at least one functional fragment comprising the domain necessary for the dimerisation of the protein P, of the mature sequence of the said homo- or heterodimeric protein P,
- at least one stop codon,
- a second ribosome binding site,
- a second sequence A as defined above,
- a sequence B comprising a nucleic acid sequence coding for an NH₂-terminal fragment of the mature homo- or heterodimeric protein,
- a sequence E consisting of a nucleic acid sequence coding for the chain of the said immunoglobulin not coded by the sequence C above,
- at least one stop codon,
- and a transcription terminator,
the entirety of these fragments being in a reading frame and coding for the complex hybrid protein according to any one of the Claims 1 to 5, displaying simultaneously the properties of the enzyme and certain properties of the Fab fragments of the immunoglobulin.

11. Transcription unit according to Claim 10, characterized in that the said homo- or heterodimeric protein is an alkaline phosphatase.

12. Transcription unit according to Claim 11, characterized in that it is composed of:
- an appropriate promoter,
- a first ribosome binding site (Shine-Dalgarno sequence),
- a first sequence A comprising the signal sequence of the structure gene of the alkaline phosphatase,
- a fragment B coding for the 6 N-terminal amino-acids of the mature alkaline phosphatase,
- a sequence C consisting of a nucleic acid sequence chosen from the group consisting of either a sequence coding for the variable region and the C_{H}1 domain of the heavy chain of an immunoglobulin, or a sequence coding for the light chain of an immunoglobulin,
- a sequence D composed of a fragment coding for the remaining 444 C-terminal amino-acids of the mature sequence of the said dimeric enzyme,
- at least one stop codon,
- a second ribosome binding site,
- a second sequence A as defined above,
- a fragment B coding for the 6 N-terminal amino-acids of the mature alkaline phosphatase,
- a nucleic acid sequence E as defined above, coding for the chain of the said immunoglobulin not coded by the sequence C above,
- at least one stop codon,
- and a transcription terminator.

13. Transcription unit according to Claim 12, characterized in that it also comprises, in particular to allow the insertion of the fragments coding for the immunoglobulins and to put the entirety of these fragments into reading frame, upstream of and/or downstream of the sequence C and upstream of and/or downstream of the sequence E, an appropriate nucleic acid fragment, this transcription unit, in reading frame, coding for a hybrid protein according to any one of the Claims 1 to 5.

14. Transcription unit according to Claim 13, characterized in that it comprises a first signal sequence A of the structure gene of the alkaline phosphatase, a first sequence B coding for the 6 N-terminal amino-acids of the alkaline phosphatase, a restriction site Bgl II, a sequence C chosen from among the group consisting of either a sequence coding for the variable region and the C_{H}1 domain of a heavy chain of an immunoglobulin, or the sequence coding for the light chain of an immunoglobulin, a restriction site Sal I, a fragment D coding for the remaining 444 C-terminal amino-acids of the alkaline phosphatase, a second sequence A, a second sequence B, a restriction site Sac I, a sequence E coding for the sequence not coded by sequence C, a restriction site Xba I, at least one stop codon and a transcription terminator.

15. Transcription unit T2, characterized in that it comprises:
- an appropriate promoter,
- a ribosome binding site (Shine-Dalgarno sequence),
- a first signal sequence A of a structure gene of a protein P₁, a sequence D able to code for a functional fragment of the mature sequence of a dimeric protein P and one or more unique restriction sites situated at the junction between the first sequence A and the sequence D and able to receive a sequence C chosen from among the group consisting of either a sequence coding for the variable region and the C_{H}1 domain of a heavy chain of an immunoglobulin or the sequence coding for the light chain of an immunoglobulin,
- at least one stop codon,
- a ribosome binding site (Shine-Dalgarno sequence),
- a second sequence A as defined above and one or more unique restriction sites situated downstream of the said sequence A and capable of receiving a sequence E coding for the sequence not coded by the sequence C,
- at least one stop codon,
- and a transcription terminator.

16. Transcription unit T2 according to Claim 15, characterized in that it comprises:
- an appropriate promoter,
- a ribosome binding site (Shine-Dalgarno sequence),
- a first signal sequence A of a structure gene of a protein P₁,
- a first sequence B coding for an NH₂-terminal fragment of a mature protein P₁, a sequence D able to code for a mature protein P or a fragment of the latter and one or more unique restriction sites situated at the junction between the NH₂-terminal fragment of the sequence coding for the protein P₁ and the sequence coding for the protein P and able to receive a sequence C as defined above,
- at least one stop codon,
- a ribosome binding site (Shine-Dalgarno sequence),
- a second sequence A as defined above,
- a second sequence B as defined above and one or more unique restriction sites situated downstream of the said sequence A and able to receive a sequence E as defined above,
- at least one stop codon,
- and a transcription terminator.

17. Transcription unit according to Claim 16, characterized in that P₁ and P are identical.

18. Transcription unit according to any one of the Claims 6 to 16, characterized in that the promoter is chosen from among the group comprising the promoter of the gene phoA and any other promoter.

19. Expression and/or cloning vectors of a complex hybrid protein according to any of the Claims 1 to 5, characterized in that each vector includes a transcription unit T1 according to any of the Claims 6 to 14 or Claim 18, inserted into an appropriate genetic structure, in particular chosen from among the group comprising the appropriate plasmids, phages, cosmids or chromosomes.

20. Vector according to Claim 19, characterized in that the genetic structure is a plasmid and the transcription unit T1 comprises a gene or a gene fragment coding for a protein P₁, identical to protein P, the said protein being a dimeric enzyme.

21. Potential expression and/or cloning vectors of a complex hybrid protein according to any of the Claims 1 to 5, characterized in that each vector includes a transcription unit T2 according to any of the Claims 15 to 18, inserted into an appropriate genetic structure, in particular chosen from among the group comprising the appropriate plasmids, phages, cosmids or chromosomes.

22. Micro-organism obtained by genetic transformation, characterized in that it is obtained by appropriate modification of a strain of *E. coli* by a vector according to any one of the Claims 19 to 21, and in particular by transformation.

23. Micro-organism according to Claim 22, characterized in that it is advantageously a strain CC118 of *E. coli* transformed by a non-loaded vector according to Claim 21.

24. Micro-organism according to Claim 22 or Claim 23, characterized in that it was deposited on 31st January 1992 under Number I-1168 at the National Collection of Micro-organism Cultures maintained by the Pasteur Institute.

25. Diagnostic reagent, characterized in that it consists of a complex hybrid protein according to any one of the Claims 1 to 5.

26. Method for the immuno-enzymatic determination of proteins, characterized in that it consists of:
- placing a suitable biological sample in contact with a diagnostic reagent according to Claim 25, and
- detecting the said diagnostic reagent in the form of a complex and/or in the free form by an appropriate colorimetric reaction.

27. Ready-to-use kit for implementing the immuno-enzymatic analysis according to Claim 25, characterized in that it comprises, in addition to the appropriate amounts of buffers and reagents useful for implementing the analysis, appropriate quantities of the reagent according to Claim 25.

28. Agent for therapeutic purposes, characterized in that it consists of a complex hybrid protein according to any one of the Claims 1 to 5.

29. Therapeutic agent according to Claim 28, characterized in that the protein P is a cytotoxic substance and the fragments of immunoglobulin associated in the base units are of human origin and display specificity towards a previously selected target cell.

30. Method for production of a bivalent and bi-specific antibody, characterized in that:
- two transcription units T1 whose parts coding for the immunoglobulin domains are different are produced in a vector according to any one of the Claims 19 and 20, these units being under the control of the same promoter and of the same transcription terminator, and then, if necessary,
- the hybrid products for which the base unit U is different to the base unit U' are selected, in particular by affinity chromatography.
